# EUROPEAN PATENT SPECIFICATION

(11) **EP 1 978 984 B1**
(45) Date of publication and mention of the grant of the patent: **10.06.2015**
(21) Application number: 07722780.9
(22) Date of filing: 31.01.2007
(51) Int. Cl.: A23K 1/00, A23L 1/302, A23L 1/304, A23L 1/30, A23L 1/305, A23K 1/16, A23K 1/18, A23L 1/29

(54) **NUTRITIONAL SYSTEM AND METHODS FOR INCREASING LONGEVITY**
ERNÄHRUNGSSYSTEM UND VERFAHREN FÜR EIN LÄNGERES LEBEN
SYSTEME NUTRITIONNEL ET PROCEDES PERMETTANT D'AUGMENTER LA LONGEVITE

(30) Priority: 01.02.2006 US 764056 P
(43) Date of publication of application: 15.10.2008
(73) Proprietor: Nestec S.A., 1800 Vevey (CH)
(72) Inventor: PAN, Yuanlong, Chesterfield, MO 63005 (US); MIDDLETON, Rondo, P., Creve Couer, MO 63141 (US); HANNAH, Steven, S., Chesterfield, MO 63017 (US)
(74) Representative: Rupp, Christian
(86) International application number: PCT/EP2007/000837
(87) International publication number: WO 2007/088046

(56) References cited:
- EP-A- 1 637 041
- WO-A-2004/026287
- CA-A1- 2 546 464
- DE-A1- 10 326 822
- US-A1- 2002 182 196
- US-A1- 2005 147 648
- US-A1- 2006 116 334
- DATABASE WPI Week 200673 Derwent Publications Ltd., London, GB; AN 2006-708988 XP002466168 & WO 2006/103750 A (NIPPON MEAT PACKERS INC) 5 October 2006 (2006-10-05)

## Description

### FIELD OF THE INVENTION

This invention relates to the field of nutritional support of health and longevity in animals. In particular, the invention provides dietary formulations and methods to mimic the physiological, biochemical and gene expression effects of calorie restriction without altering dietary intake.

### BACKGROUND OF THE INVENTION

Restriction of caloric intake well below *ad libitum* levels has been shown to increase lifespan, reduce or delay the onset of many age-related conditions, improve stress resistance and decelerate functional decline in numerous animal species, including mammals such as rodents and primates (*see, e.g.,* D.K. Ingram et al. (2004) Ann. N.Y. Acad. Sci. 1019: 412-423). Indeed, clinical trials have been initiated to evaluate the longevity-promoting effect of caloric restriction (CR) in humans. But in humans and animals alike, it seems unlikely that CR is a viable strategy for increasing longevity in most individuals, due to the degree and length of restriction required. For this reason, research has focused on the identification of substances, *e.g.,* pharmaceutical agents, nutritional substances and the like, capable of mimicking the effect of CR without a substantive change in dietary intake.

Efforts have been directed toward identifying agents that can mimic one or more of the physiological or biochemical effects of CR (*see, e.g.,* Ingram et al., 2004, *supra*), or that can mimic the gene expression profile associated with CR in certain tissues and organs (*e.g.,* Spindler, U.S. Patent 6,406,853; U.S. Patent Publication No. 2003/0124540). In connection with the latter, methods purported to analyze genes associated with CR and to screen for CR mimetics based on gene expression profiling have been described (Spindler et al., U.S. Patent Publication Nos. 2004/0180003, 2004/0191775 and 2005/0013776).

For example, CR has been observed to have one or more of the following effects in various studies: (1) reduction in oxidative stress and oxidative damage (*e.g.,* Weindruch, Scientific American Jan. 1996, 46-52); (2) reduction in glycation damage (Novelli et al. (1998), J. Gerontol. A. Biol. Sci. Med. Sci. 53: B94-101); (3) decrease in body weight and body fat content (Bertrand et al. (1980), J. Gerontol. 35:827-835); (4) increase in insulin sensitivity and reduction in blood glucose and blood insulin levels (Lane et al. (1995), Am. J. Physiol. 268: E941-E948; Kemnitz et al. (1994), Am. J. Physiol. 266:E540-E547); and (5) reduction in chronic inflammation (Chung et. Al. (2002), Microsc. Res. Tech. 59:264-272. In this regard, it has been reported that administration of long-chain free fatty acids, such as palmitic acid and oleic acid, and their CoA derivatives, might mimic the effect of CR in one or more biochemical parameters (Chacon, U.S. Patent Publication No. 2002/0173450). Carnosine (beta-alanyl-L-histidine) is reported to be present in long-lived tissues and purported to delay aging through its function as an antioxidant, free radical scavenger and anti-glycation agent (Hipkiss (1998), Int. J. Cell Biol. 30: 863-868; Hipkiss & Brownson (2000), Cell Mol. Life Sci. 57: 747-753).

Pitha et al., (U.S. Patent Publication No. 2002/0035071) reported that a beneficial biological result associated with CR could be obtained by administering an agent that blocks metabolism of glucose, such as 2-deoxy-D-glucose, 5-thio-D-glucose, mannoheptulose, 3-O-methylglucose, 1-5-anhydro-D-glucitol or 2,5-anhydro-D-mannitol.

Malnoe et al. (WO 02/071874; U.S. Patent Publication No. 2005/0100617) described a food composition for administration to mammals that was purportedly able to mimic the effects of CR on gene expression. The composition contained an antioxidant and a substance that stimulates energy metabolism, such as carnitine or a carnitine derivative.

Young et al. (WO 01/17366) described a method for increasing the longevity of elderly pets by administration of a nutritional composition containing a calcium source, an antioxidant and, optionally, a pre-biotic or probiotic microorganism, a source of zinc and glutamine.

Cupp et al., (U.S. Patent Publication 2005/0123643) also described a method for improving the longevity of elderly pets by administering a nutritional composition containing an oil blend, an antioxidant, a source of linoleic acid and, optionally, a prebiotic such as inulin or fructooligosaccharides.

US 2005/0116334 describes formulations for the prevention and treatment of cardiovascular diseases, arthrosclerosis, and risk factors thereof.

EP 1 637 041 A1 is directed to methods and compositions for improving the longevity, activity, and health of elderly pets.

WO 2004/026287 discloses orally administrable composition for improving skin quality.

DE 103 26 822 A1 is directed to a supplement for improving the productivity, concentration and endurance.

US 2005/147648 discloses zeaxanthin formulations with additional ocular-active nutrients, for protecting eye health and treating eye disorders.

US 2002/0182196 A1 is directed to a composition and method for normalizing impaired or deteriorating neurological function.

CA 2 546 464 describes a method of treating aged and wrinkled skin.

WO 2006/103750 relates to a composition, functional food and pharmaceutical composition for improvement in obesity.

WO 2004/100896 describes an anti-aging nutritional supplement.

US 2004/0047896 relates to a food composition intended to prevent or restore age-related functional deficits in mammals.

Calder reports on the relation between n-3 polyunsaturated fatty acids and inflammation ("n-3 polyunsaturated fatty acids and inflammation: from molecularbiology to the clinic", Lipids, 2003, vol. 38, no. 4, pp. 343-352).

Hipkiss provides a review on the potential impacts of carnivorous diets ("Glycation, ageing and carnosine: Are carnivorous diets beneficial" in Mechanism of Ageing and Development, 2005, vol. 126, pp. 1034-1039).

Despite the availability of the methods and agents described above, there remains a need for methods and compositions that can mimic the effects of CR without requiring individuals to substantially modify their caloric intake.

### SUMMARY OF THE INVENTION

The invention is directed to a dietary formulation comprising five categories of ingredients that improve longevity by mimicking at least one longevity-promoting effect of caloric restriction, wherein the categories are:
(a) antioxidants;
(b) anti-glycation agents;
(c) reducers of body weight or body fat;
(d) promoters of high insulin sensitivity or low blood insulin or blood glucose; and
(e) anti-inflammatory agents.

The antioxidants can be water-soluble; the water-soluble antioxidants cab include one or more of Vitamin C, polyphenols, proanthocyanidins, anthocyanins, bioflavonoids, a source of selenium, alpha-lipoic acid, glutathione, catechin, epicatechin, epigallocatechin, epigallocatechin gallate, epicatechin gallate or cysteine; the source of selenium can be at least one of sodium selenite, sodium selenate or L-selenomethionine.

The antioxidants can be fat-soluble; the fat-soluble antioxidants can include one or more of Vitamin E, gamma tocopherol, alpha-carotene, beta-carotene, lutein, zeaxanthin, retinal, astaxanthin, cryptoxanthin, natural mixed carotenoids, lycopene or resveratrol.

The formulation can contain fat-soluble and water-soluble antioxidants; the antioxidants can include Vitamin E, Vitamin C, natural carotenoids, a source of selenium, and lycopene.

The anti-glycation agents can include one or more of carnosine or aminoguanidine.

The reducers of body weight or body fat can include one or more of conjugated linoleic acid, L-carnitine, acetyl-L-carnitine, pyruvate, polyunsaturated fatty acids, medium chain fatty acids, medium chain triglycerides, or soy isoflavones and their metabolites.

The promoters of high insulin sensitivity or low blood insulin or blood glucose can include one or more of a source of chromium, cinnamon, cinnamon extract, polyphenols from cinnamon and witch hazel, coffee berry extract, chlorogenic acid, caffeic acid, a source of zinc, or grape seed extract.

The anti-inflammatory agents can include one or more of a source of omega-3 fatty acids or a source of curcumin; optionally wherein (a) the source of omega-3 fatty acid is at least one of α-linolenic acid, eicosapentaenoic acid, docosapentaenoic acid, docosahexaenoic acid, flax seed, flax oil, walnuts, canola oil, wheat germ, or fish oil: or (b) the source of curcumin is (1,7-bis-(4-hydroxy-3-methoxyphenyl)-hepta-1,6-diene-3,5-dione; 1-(4-hydroxyphenyl)-7-(4-hydroxy-3-methoxyphenyl)-hepta-1,6-diene-3,5-dione; 1,7-bis-(4-hydroxyphenyl)-hepta-1,6-diene-3,5-dione), demethoxycurcumin, or bisdemethoxycurcumin.

### BRIEF DESCRIPTION OF THE DRAWINGS

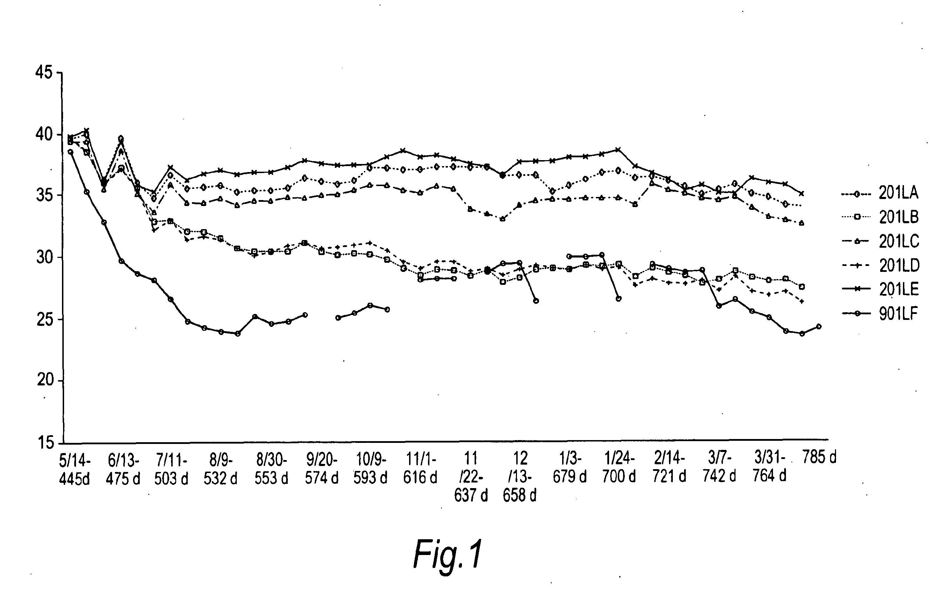

**Figure 1** shows weights of animals subjected to diets or CR. Middle-aged male mice (C57B1/6, 15 mice per group) were fed 24 grams/week control (201LE) or test diets (201LA = cocktail 1, 201LB = cocktails I + II, 201LC = cocktails I + III, and 201LD = cocktail I+II+III) or 18 grams/week caloric restriction (CR) diet (901LF). After 11 months of feeding, mice maintained on two test diets containing cocktail II (201 LB and 201LD) reduced their body weights to a level comparable to those of mice maintained on the CR diet, without reduction in food intake.

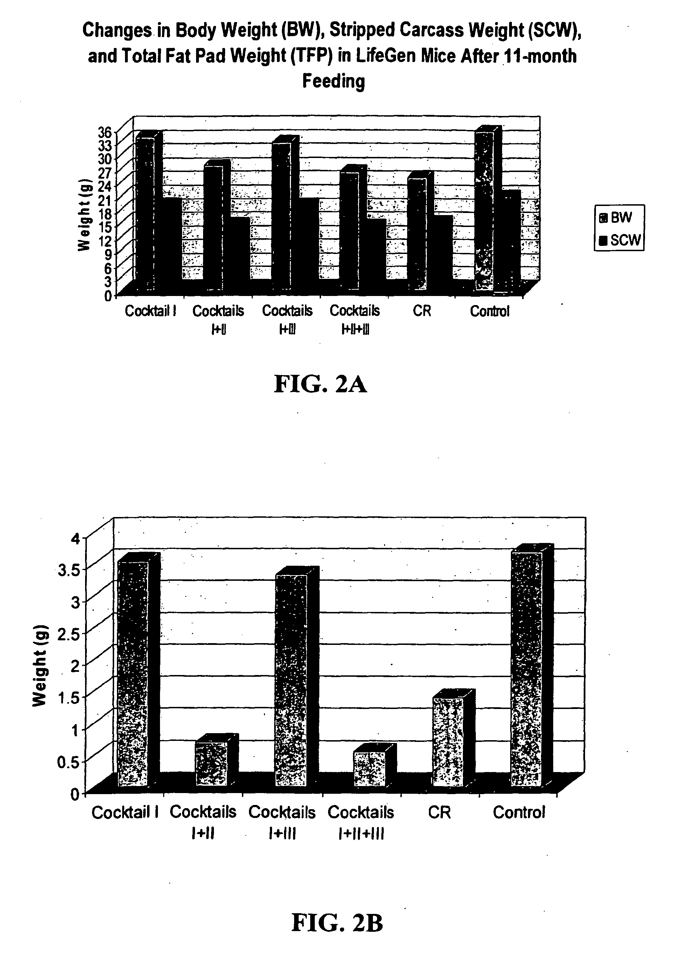

**Figure 2** shows changes in body weight (BW), stripped carcass weight (SCW) or total fat pad weight in animals subjected to diets or caloric restriction. Middle-aged male mice (C57B/L6, 15 mice per group) were fed 24 grams/week control (201LE) or test diets (201 LA = cocktail 1, 201LB = cocktails I + II, 201LC = cocktails I + III, and 201LD = cocktail I+II+III) or 18 grams/week caloric restriction (CR) diet (901LF). After 11 months of feeding, mice maintained on two test diets containing cocktail II (201LB and 201LD) had body weight and stripped carcass weights comparable to those of CR mice (top panel), while the total fat pad weights of the mice maintained on two test diets containing cocktail II (201 LB and 201LD) were 50% less than those of CR mice (bottom panel).

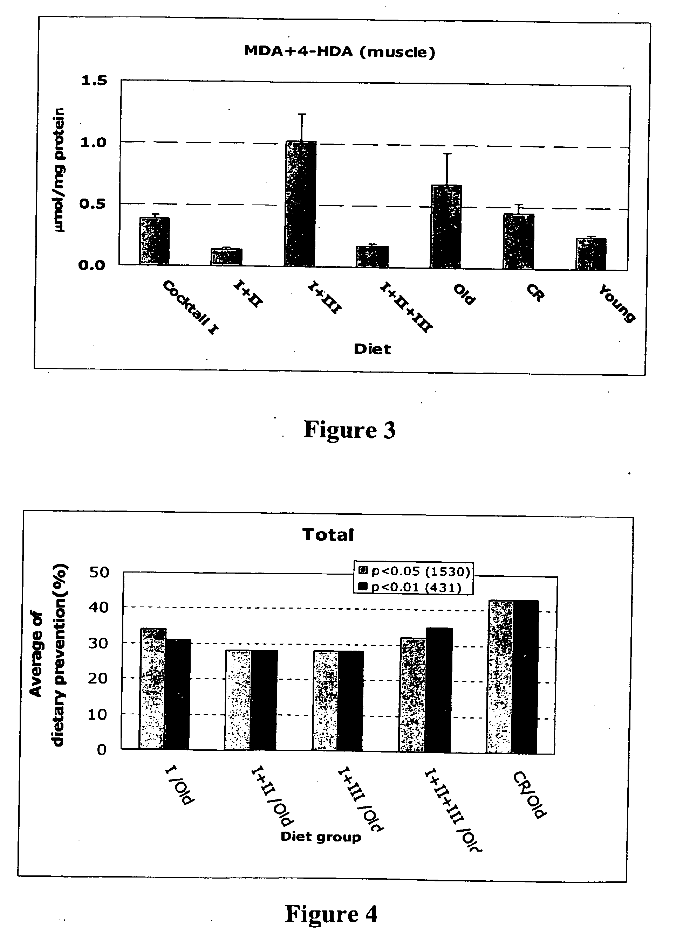

**Figure 3** shows concentration of malonyldialdehyde (MDA) and 4-hydroxyalkenals (4-HDA) in animals fed respective diets or subjected to CR. Middle-aged male mice (C57B/L6, 15 mice per group) were fed 24 grams/week control (201LE) or test diets (201 LA = cocktail 1, 201 LB = cocktails I + II, 201 LC = cocktails I + III, and 201LD = cocktail I+II+III) or 18 grams/week caloric restriction (CR) diet (901LF). After 11 months of feeding, muscle lipid peroxidation products (MDA and 4-HDA) were the highest in the mice fed test diet containing cocktails I + III, followed by old mice fed control diet. The test diet containing cocktail I alone had reduced MDA and 4-HDA comparable to those of CR mice. Two test diets (201 LB and 201LD) further reduced muscle MDA and 4-HDA to levels lower than those of young mice.
**Figure 4** shows anti-aging effect (% as compared to control) on gene expression. Middle-aged male mice (C57B1/6, 15 mice per group) were fed 24 grams/week control (201LE) or test diets (201LA = cocktail 1, 201LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. The average anti-aging effects were calculated for each test diets and CR. For instance, with p value less than 0.01, a total of 431 genes were affected by aging, and CR prevented these aging-induced gene expression changes by an average of 43%. The nutrient cocktails I, I+II, I+III, and I+II+III prevented these aging-induced gene expression changes by an average of 29, 27, 24 and 30%, respectively. Similar anti-aging effects were observed in both CR and nutrients at p < 0.05 with a total of 1530 genes affected by aging.

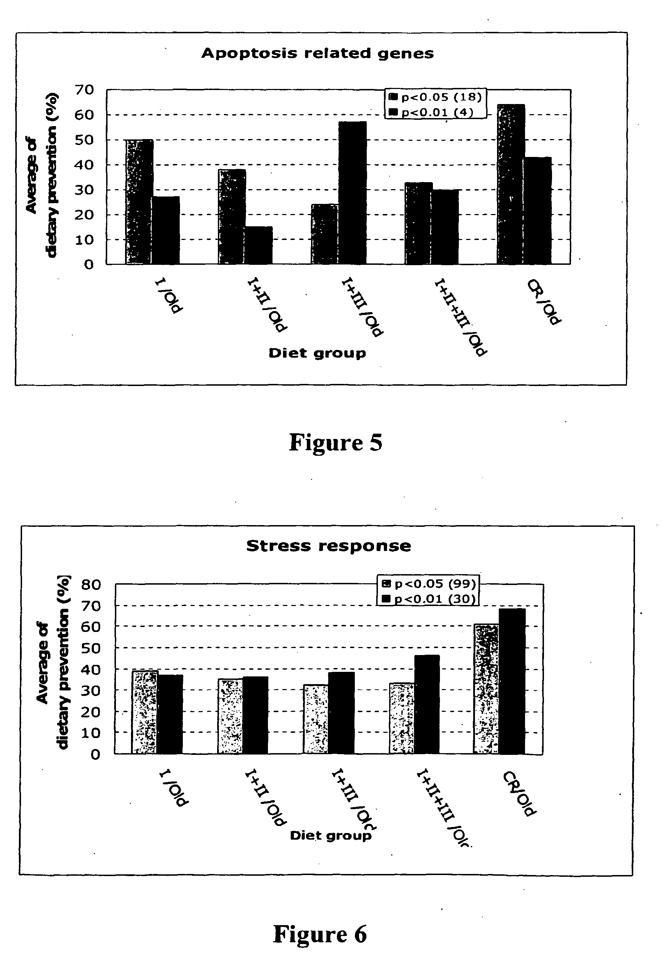

**Figure 5** shows anti-aging effect (% as compared to control) on apoptosis-related gene expression. Middle-aged male mice (C57B1/6, 15 mice per group) were fed 24 grams/week control (201LE) or test diets (201LA = cocktail 1, 201LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. The average anti-aging effects on aging-affected genes involved in apoptosis were calculated for each test diets and CR at p < 0.01 or 0.05.
**Figure 6** shows anti-aging effect (% as compared to control) on stress response-gene expression. Middle-aged male mice (C57B1/6, 15 mice per group) were fed 24 grams/week control (201LE) or test diets (201 LA = cocktail 1, 201LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. The average anti-aging effects on aging-affected genes involved in stress response were calculated for each test diets and CR at p < 0.01 or 0.05.

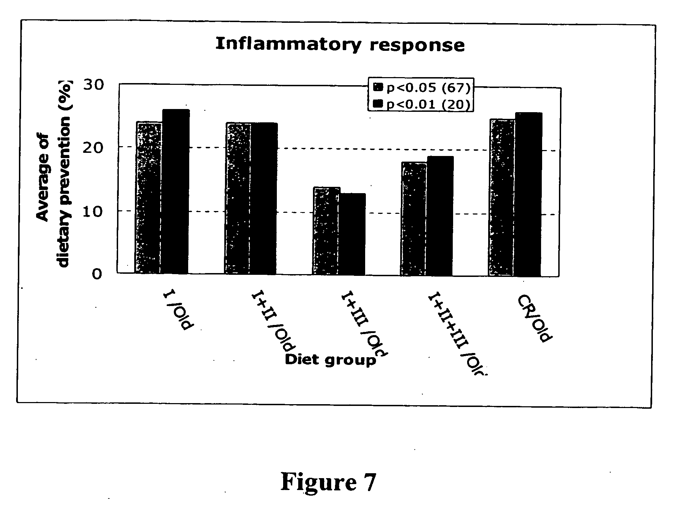

**Figure 7** shows anti-aging effect (% as compared to control) on inflammatory response gene expression. Middle-aged male mice (C57B1/6, 15 mice per group) were fed 24 grams/week control (201LE) or test diets (201 LA = cocktail 1, 201 LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. The average anti-aging effects on aging-affected genes involved in inflammatory response were calculated for each test diets and CR at p < 0.01 or 0.05.

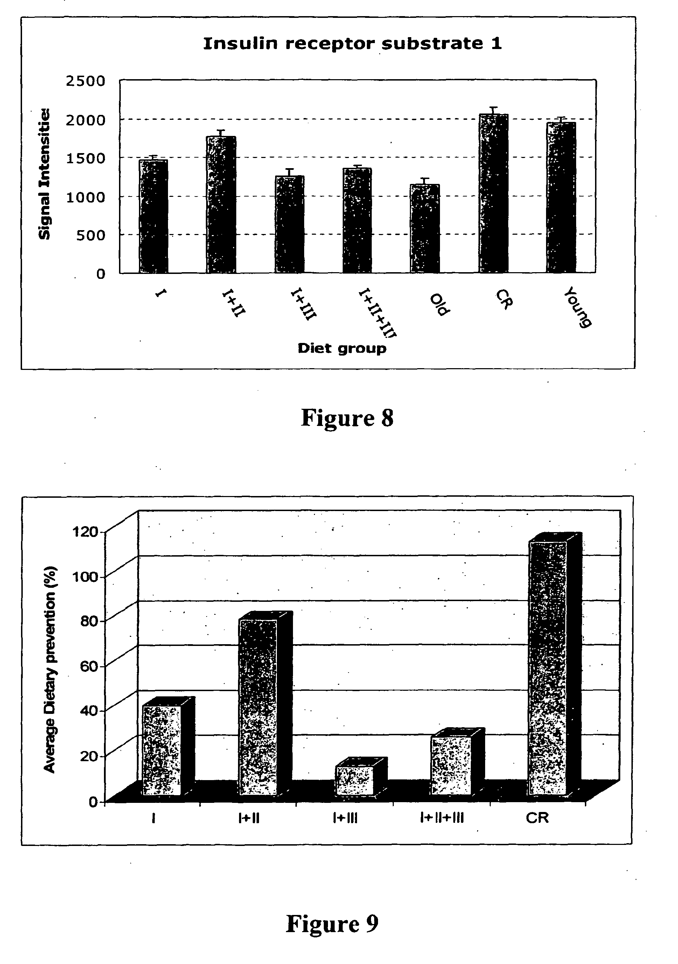

**Figure 8** shows microarray signal intensities for expression of insulin receptor substrate-1 gene expression. Middle-aged male mice (C57B1/6, 15 mice per group) were fed 24 grams/week control (201LE) or test diets (201 LA = cocktail 1, 201 LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. IRS-1 signal intensities were determined in the microarray for mouse muscle tissue in mice fed each of the cocktail diets and in mice fed a caloric restriction dietary regimen, and were compared to IRS-1 signal intensities in muscle tissue from control young and old mice.
**Figure 9** shows anti-aging effect (% as compared to control) on insulin receptor substrate 1 gene expression. Middle-aged male mice (C57B1/6, 15 mice per group) were fed 24 grams/week control (201LE) or test diets (201 LA = cocktail 1, 201 LB = cocktails I + II, 201LC = cocktails I + III, and 201LD = cocktail I+II+III) or 18 grams/week caloric restriction (CR) diet (901 LF). After 11 months of feeding, gene expression profiles of young mice, old mice, CR mice and mice fed four test diets were analyzed with Affymetrix mouse 430A GeneChip® array. The average prevention effects on aging-induced reduction of IRS-1 were calculated for each test diets and CR at p < 0.01. CR completely (100%) prevented aging-induced reduction of IRS-1 gene expression in skeletal muscle, followed by cocktail I+II (78%).

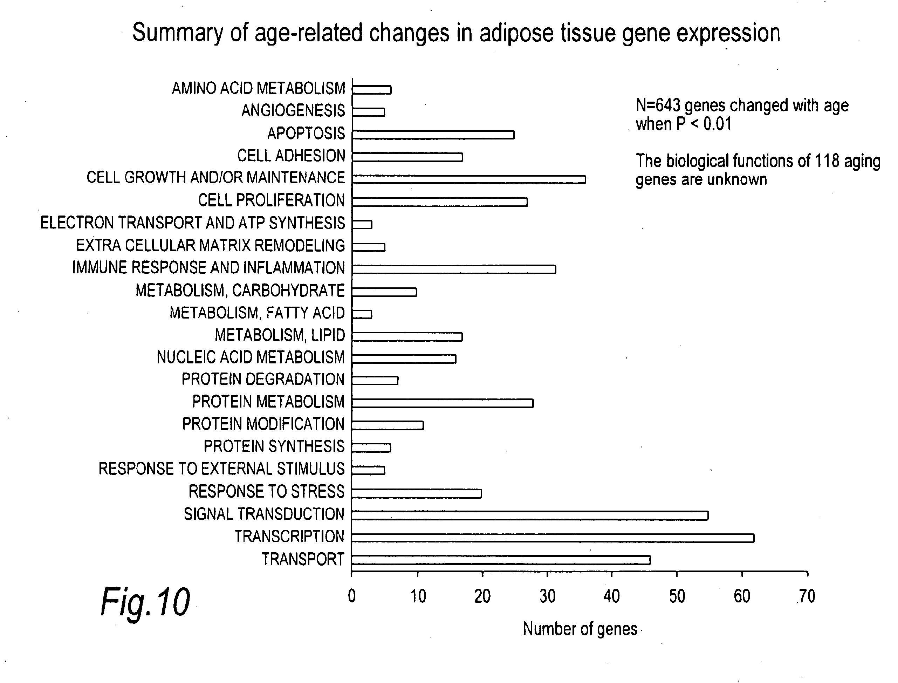

**Figure 10** shows a summary of age-related changes in adipose tissue gene expression. Middle-aged male mice (C57B1/6, 15 mice per group) were fed 24 grams/week control (201LE) or test diets (201LA = cocktail 1, 201LB = cocktails I + II, 201LC = cocktails I + III, and 201LD = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. Age-induced changes in gene expression of mouse white adipose tissue are summarized.

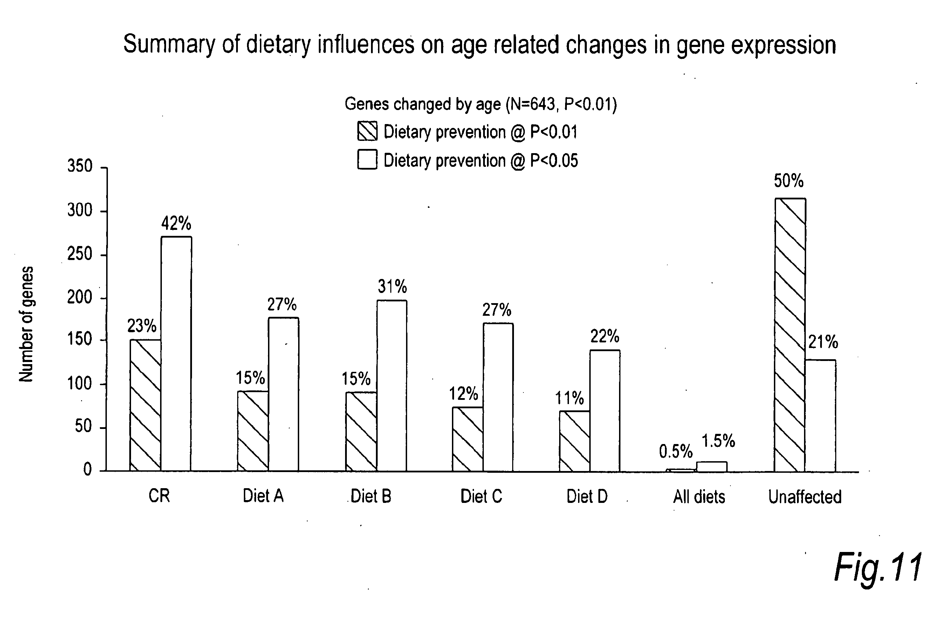

**Figure 11** shows a summary of dietary influences on age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of young mice, old mice, CR mice and mice fed four test diets were analyzed with Affymetrix mouse 430A GeneChip® array. The percentages of aging-affected genes in mouse white adipose tissue that were retarded by CR or nutrient cocktails are shown. At p<0.01, CR retarded 23% of the aging-affected genes, followed by cocktail I and cocktails I+II (15%). At p<0.05, CR retarded 42% of the aging-affected genes, followed by cocktails I + II (31 %), cocktail I (27%), cocktails I + III (27%), and cocktails I+II+III (22%). All test diets commonly retarded 0.5 (p<0.01) to 1.5% (p<0.05) of the aging-affected genes.

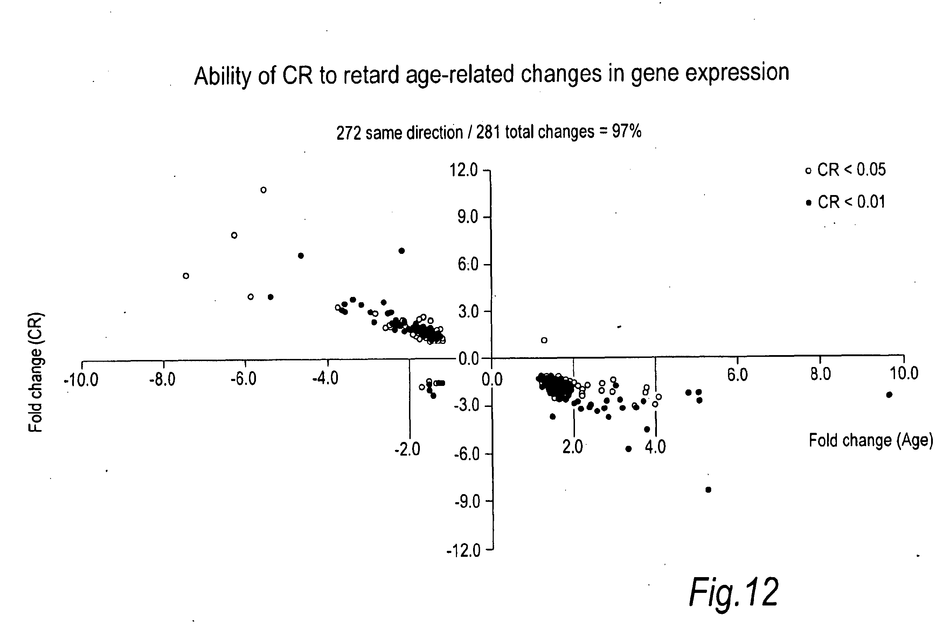

**Figure 12** is a scatter plot showing the ability of caloric restriction (CR) to retard age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes were significantly changed with age at P< 0.01. Of this set of "aging genes", 281 genes were changed with calorie restriction (CR) at P<0.05, and CR prevented the age-associated change in 272 of the 281 genes. In the plot, the x-axis represents the fold change with age and the y-axis represents the fold change with CR. Dark circles represent genes where the change in expression with CR was significant at P < 0.01; light circles represent genes where the change in expression with CR was significant at P < 0.05.

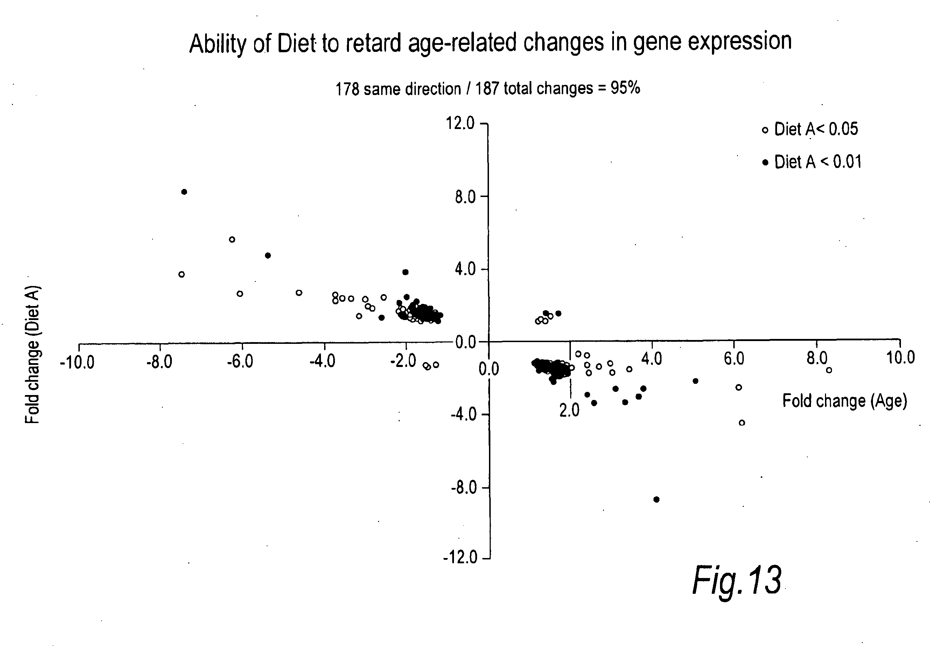

**Figure 13** is a scatter plot showing the ability of Diet A to retard age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes that were significantly changed with age at P< 0.01. Of this set of "aging genes", 187 genes were changed with Diet A at P<0.05, and Diet A prevented the age-associated change in 178 of the 187 genes. In the plot, the x-axis represents the fold change with age and the y-axis represents the fold change with Diet A. Darkcircles represent genes where the change in expression with Diet A was significant at P < 0.01; light circles represent genes where the change in expression with Diet A was significant at P < 0.05.

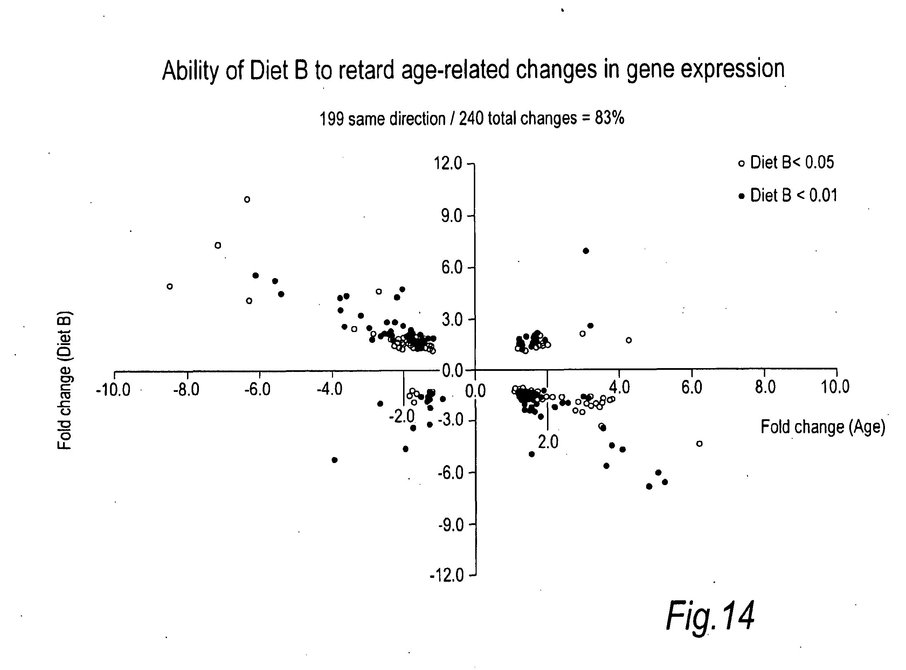

**Figure 14** is a scatter plot showing the ability of Diet B to retard age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes were significantly changed with age at P< 0.01. Of this set of "aging genes", 240 genes were changed with Diet B at P<0.05, and Diet B prevented the age-associated change in 199 of the 240 genes. In the plot, the x-axis represents the fold change with age and the y-axis represents the fold change with Diet B. Dark circles represent genes where the change in expression with Diet B was significant at P < 0.01; light circles represent genes where the change in expression with Diet B was significant at P < 0.05.

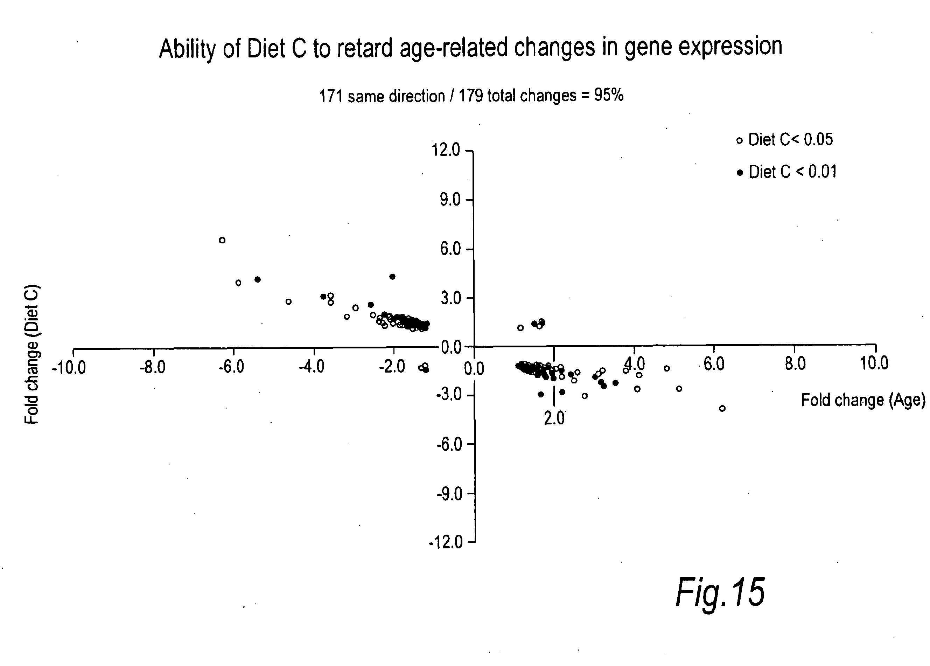

**Figure 15** is a scatter plot showing the ability of Diet C to retard age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes were significantly changed with age at P< 0.01. Of this set of "aging genes", 179 genes were changed with Diet C at P<0.05, and Diet C prevented the age-associated change in 171 of the 179 genes. In the plot, the x-axis represents the fold change with age and the y-axis represents the fold change with Diet C. Dark circles represent genes where the change in expression with Diet C was significant at P < 0.01; light circles represent genes where the change in expression with Diet C was significant at P < 0.05.

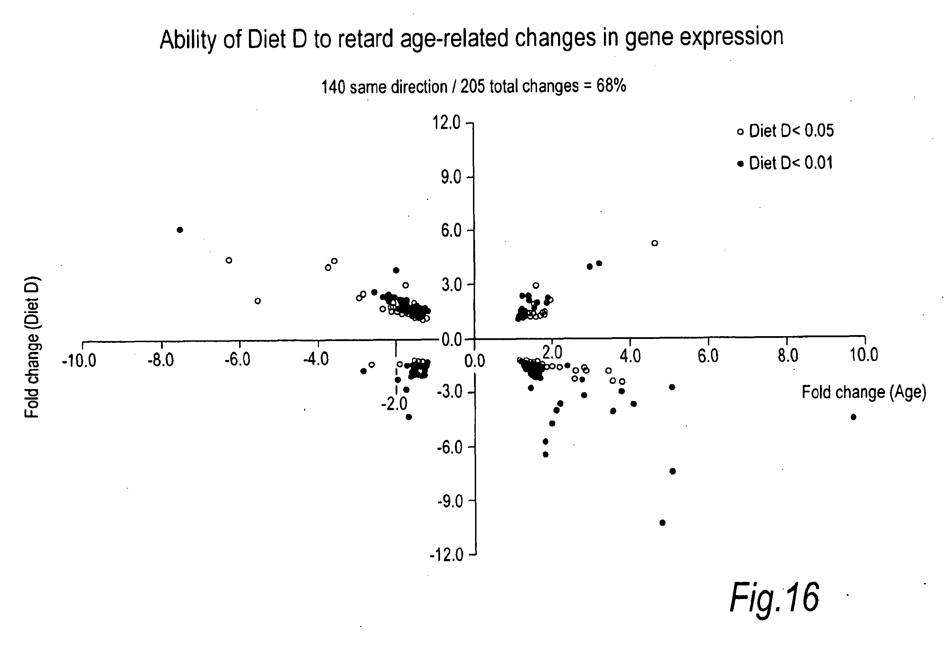

**Figure 16** is a scatter plot showing the ability of Diet D to retard age-related changes in gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes were significantly changed with age at P< 0.01. Of this set of "aging genes", 205 genes were changed with Diet D at P<0.05, and Diet D prevented the age-associated change in 140 of the 205 genes. In the plot, the x-axis represents the fold change with age and the y-axis represents the fold change with Diet D. Dark circles represent genes where the change in expression with Diet D was significant at P < 0.01; light circles represent genes where the change in expression with Diet D was significant at P < 0.05.

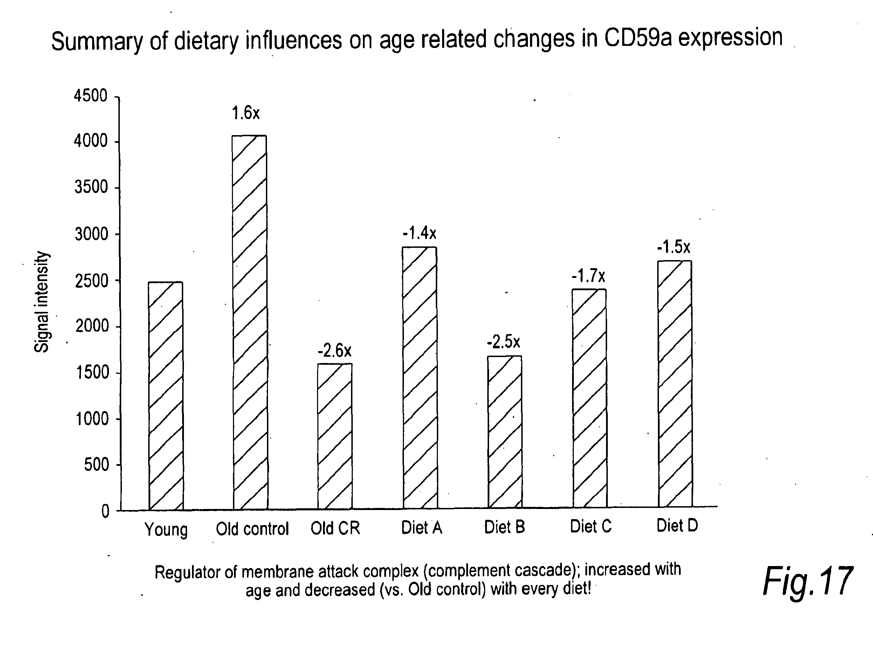

**Figure 17** shows a summary of dietary influences on age-related changes in CD59a gene expression. Middle-aged male mice (C57B1/6, 15 mice per group) were fed 24 grams/week control (201LE) or test diets (Diet A = cocktail 1, diet B = cocktails I + II, diet C = cocktails I + III, and diet D = cocktail I+II+III) or 18 grams/week caloric restriction (CR) diet (901LF). After 11 months of feeding, gene expression profiles of white adipose tissue from young mice, old mice, CR mice and mice fed four test diets were analyzed with Affymetrix mouse 430A GeneChip® array. A total of 643 genes were significantly changed with age at P< 0.01. Aging-induced increase in CD59a gene expression was retarded by CR and all test diets.

### DETAILED DESCRIPTION

Within this specification s have been described in a way which enables a clear and concise specification to be written, but it is intended and will be appreciated that s may be variously combined or separated without parting from the invention.

The terms "functional ingredient, "functional agent" or "functional component" as used interchangeably herein refer to substances known to have a functional feature or activity in one or more of the following categories: (1) reducing oxidative stress or damage; (2) anti-glycation agent; (3) reducing body weight, especially body fat; (4) stimulating insulin sensitivity or reducing blood glucose or blood insulin; and (5) anti-inflammatory agent.

"Effective amount" refers to an amount of a compound, material, or composition, as described herein that is effective to achieve a particular biological result. Such results include, but are not limited to, improving age-compromised factors, increasing longevity, reducing the incidence and/or delaying the onset of age-related diseases, reducing functional decline, and improving the biochemical, molecular, cellular, physiological, and phenotypical effects of aging. Such effective activity may be achieved, for example, by administering the compositions of the present invention to an individual.

A "subject" or "individual" refers to an animal of any species. In various s, the animal is a mammal, and may be a human.

As used herein, a "dietary supplement" is a product that is intended to be ingested in addition to the normal diet of an animal. The animal is a mammal, and may be a human

As used herein, a "food product formulated for human consumption" is any composition intended for ingestion by a human being.

As used herein, the term "pet food" or "pet food composition" means a composition that is intended for ingestion by an animal, and preferably by companion animals. A "complete and nutritionally balanced pet food," is one that contains all known required nutrients in appropriate amounts and proportions based on recommendations of recognized authorities in the field of companion animal nutrition, and is therefore capable of serving as a sole source of dietary intake to maintain life or promote production, without the addition of supplemental nutritional sources. Nutritionally balanced pet food compositions are widely known and widely used in the art.

"Calorie restriction" or "caloric restriction" are used interchangeably herein, and refer to any diet regimen low in calories without undernutrition. In general, the limitation is of total calories derived from of carbohydrates, fats, and proteins. The limitation is typically, although not limited to, about 25% to about 40% of the caloric intake relative to *ad libitum* consumption.

"Longevity" refers generally to the duration of life beyond the average life expectancy for a particular species. "Enhanced longevity" or "increased longevity" refers to any significant extension of the life span of a particular animal beyond the average life expectancy for the species to which the animal belongs.

"Young" refers generally to an individual in young adulthood, i.e., matured past puberty or adolescence, as would be defined by species in accordance with known parameters. "Aged" or "old," as used herein, refers to an individual who is physically or chronologically within the last 30% of its average life expectancy.

The inventors have determined that a number of the physiological, biochemical and/or gene expression features associated with CR can be mimicked through the administration of a formulation containing a combination of three or more categories of functional ingredients. Such formulations have proved to be effective in mimicking CR, as compared with previous formulations and methods focusing on only single nutrients or one or two categories of functional ingredients that failed to mimic CR benefits.

Thus, disclosed are nutritional systems which mimic the effects of caloric restriction without restricting caloric intake. The nutritional systems comprise the formulation and administration of combinations of nutrients that have various intended functions in the body, falling into three or more of the following activities; (1) antioxidant activity; (2) inhibition of glycation damage; (3) reduction of body weight, especially body fat; and (4) promotion of high insulin sensitivity and low blood insulin/glucose; and (5) anti-inflammatory activity.

When administered to animals, the nutritional systems described herein have been shown to mimic CR in various physiological and biochemical effects, including alteration in body weight and fat accumulation, reduction in lipid peroxidation, and survival rate. The inventors have also determined that, as with CR, the nutritional systems are capable of retarding, to various extents, age related changes in gene expression in bodily tissues. Accordingly, the nutritional systems described herein can provide an advantageous alternative or supplement to CR in increasing longevity.

The five intended functions can be combined in formulations comprising a combination of functional ingredients. For example, one formulation comprises at least one antioxidant, preferably one water-soluble antioxidant and one fat-soluble antioxidant. Another formulation comprises at least one functional ingredient that inhibits glycation damage, at least one functional ingredient that promotes reduction of body weight, especially body fat; and/or at least one functional ingredient for promotion of high insulin sensitivity and low blood insulin/glucose. Another formulation comprises at least one functional ingredient that reduces chronic inflammation.

The formulations can be administered to primates, including humans. Such formulations may also be administered to animals such as, but not limited to, companion animals (*e.g.,* dogs, cats, ferrets, birds), farm animals (*e.g.,* pigs, goats, sheep, cattle, horses, fowl, llamas). The compositions may also be administered to exotic animals, particularly zoo animals and endangered species. The formulation can contain at least one antioxidant, preferably one water-soluble antioxidant and one fat-soluble antioxidant. Water soluble antioxidants include, but not limited to, vitamins C, polyphenols from various berries (cranberry, blueberry, bilberry and the like), proanthocyanidins and anthocyanins from grape seeds and bark of the European coastal pine and *Pinus maritime,* bioflavonoids (taxifolin, naringenin, hesperetin, 6-hydroxyflavanone, 2'- hydroxyflavanone, 4'- hydroxyflavanone) from fruits (especially citrus fruits) and vegetables, L-selenomethionine, alpha-Lipoic Acid, glutathione, catechin, epicatechin, epigallocatechin, epigallocatechin gallate, epicatechin gallate, cysteine. Fat soluble antioxidants include, but are not limited to, vitamin E (alpha-tocopherol acetate), gamma-tocopherol, alpha-carotene, beta-carotene, lutein, zeaxanthin, retinal, astaxanthin, cryptoxanthin, natural mixed carotenoids, lycopene and resveratrol, to name a few. A formulation may include a combination of all of these antioxidants.

In the antioxidant-rich formulation, Vitamin E and/or Vitamin C may be provided to deliver about 100-1000 mg/kg of the diet. Vitamin E or Vitamin C can be provided to deliver about 200-800 mg/kg of the diet, or about 300-700 mg/kg, or about about 400-600 mg/kg, or about 450-500 mg/kg of the diet.

Carotenoids are a class of natural fat-soluble pigments found principally in plants, algae, photosynthetic and some non-photosynthetic bacteria, yeasts, and molds. About 600 different carotenoids are known to occur naturally (Ong & Tee. (1992) Meth. Enzymol., 213:142-167), and new carotenoids continue to be identified (Mercadante, A. (1999) "New carotenoids: recent progress" Invited Lecture 2. Abstracts of the 12th International Carotenoid Symposium, Cairns, Australia, July 1999). Carotenoids are defined by their chemical structure. The majority carotenoids are derived from a 40-carbon polyene chain. This chain may be terminated by cyclic end-groups (rings) as shown in Formula I below: 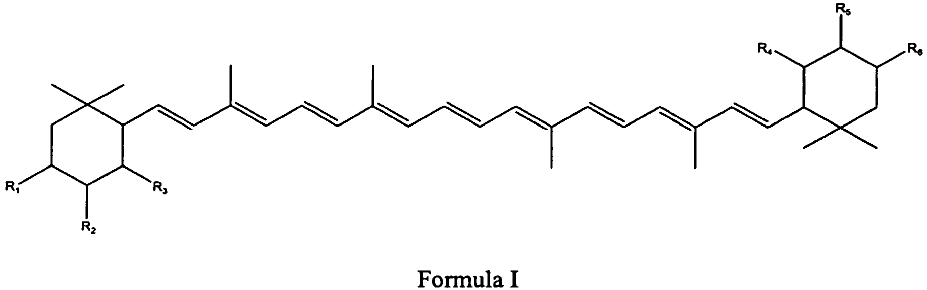 Formula I may be complemented with oxygen-containing functional groups. For example, R₁, R₃, R₄ and R₆ may be independently H or OH and R₂ and R₅ may be independently H or =O. The rings may each contain a double bond. In general, hydrocarbon carotenoids are known as carotenes, while oxygenated derivatives of these hydrocarbons are known as xanthophylls. Non-limiting examples of carotenoids are beta-carotene, zeaxanthin, astaxanthin, cryptoxanthin, and lutein.

Carotenoids can be provided to deliver about 1-100 mg/kg of the diet. Carotenoids can be provided to deliver about 10-90 mg/kg of the diet, or about 20-80 mg/kg, 30-70 mg/kg, 40-60 mg/kg, or about 50 mg/kg of the diet.

In addition to other carotenoids, the formulation may specifically include an amount of the purified carotenoid, lycopene. Lycopene is a carotene having the structure of Formula II:

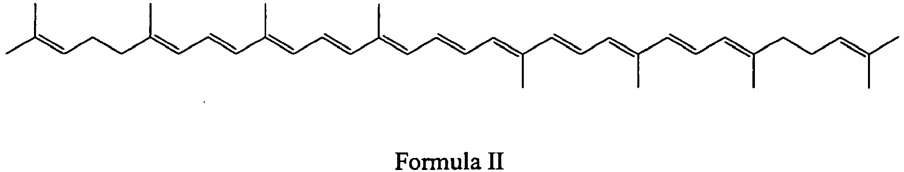

Lycopene may be provided to deliver about 1-100 mg/kg of the diet, or about 10-90, 20-80, 30-70, 40-60, or about 50 mg/kg of the diet.

An antioxidant-rich formulation may also contain a source of selenium. The trace element, selenium may be provided as inorganic selenium, such as, for example, sodium selenite or sodium selenate. L-selenomethionine ((S)-(+)-2-amino-4-(methylseleno)-butanoic acid) can be used as it is natural, stable and absorbed more readily. Typically, a source of selenium is provided to deliver about 0.01 to about 0.4 mg selenium per kilogram of the diet. Selenium can be delivered at about 0.05 to about 0.35 mg/kg of the diet, or about 0.075 to about 0.3 mg/kg, or about 0.1 to about 0.275 mg/kg, or about 0.15 to about 0.25 mg/kg, or about 0.2 mg/kg of the diet.

A formulation referred to herein as "Cocktail I" can provide the following in a diet: Vitamin E, 500 mg/kg; Vitamin C, 450 mg/kg; L-selenomethionine, 0.2 mg/kg; mixed carotenoids, 50 mg/kg; lycopene, 50 mg/kg. Cocktail I can provide the following: Vitamin E, 500 mg/day; Vitamin C, 450 mg/day; L-selenomethionine, 200 µg/day; mixed carotenoids, 2500 IU/day; lycopene, 15 mg/day.

When administered to animals, a cocktail of this type was shown to improve survival rates to levels similar to CR, without substantially affecting body weight or body composition, and to retard, to various extents, a significant percentage of age-related changes in gene expression, as described in detail in the examples.

Another type of formulation may be composed of two or three subgroups of functional ingredients, for example: (a) an inhibitor of glycation damage; (b) a reducer of body weight, especially body fat; and (c) a promoter of high insulin sensitivity and low blood insulin/glucose. Functional ingredients that inhibit glycation damage include, but are not limited to, carnosine and synthetic anti-glycation compounds such as aminoguanidine. Functional ingredients that promote reduction of body weight and body fat include, but are not limited to, pyruvate, polyunsaturated fatty acids, medium chain fatty acids, medium chain triglycerides, conjugated linoleic acid (CLA), soy isoflavones and their metabolites, L-carnitine and acetyl-L-carnitine. Functional ingredients that promote high insulin sensitivity and low blood insulin/glucose include, but are not limited to, a source of chromium, cinnamon, cinnamon extract, polyphenols from cinnamon and witch hazel, coffee berry extract, chlorogenic acid, caffeic acid, a source of zinc, and grape seed extract.

Thus, a mixed nutriment formulation can comprise at least one functional ingredient selected from each of two or three categories of functional ingredients. A mixed nutriment formulation can comprise a combination of chromium picolinate, grape seed extract, a source of zinc, conjugated linoleic acid (CLA), L-carnitine, acetyl-L-carnitine and carnosine.

Chromium picolinate may be provided in the following approximate ranges of mg/kg of the diet: about 0.1 to about 1.0, about 0.2 to about 0.9, about 0.3 to about 0.8, about 0.4 to about 0.75, about 0.45 to about 0.6, or about 0.5 mg/kg of the diet.

Formulations may also contain grape seed extract which is a source of, for example, proanthocyanidins, bioflavonoids, and catechins. Suitable amounts may comprise about 50-500, 100-400, 150-350, 200-300, or about 250 mg/kg of the diet.

Formulations may also contain a source of zinc, such as, for example, zinc chloride, zinc acetate, zinc gluconate, zinc monomethionate and zinc sulfate. The formulation can contain zinc sulfate in an amount of about 100-300, 125-275, 150-250, 175-225 or about 190 mg/kg of the diet. The formulation can contain zinc monomethionate in an amount of about 25-125, 50-100, 60-90, or about 70-80 mg/kg of the diet.

Formulations may also contain one or more ingredients that affect metabolism and promote fat loss and/or preservation of lean body mass, including conjugated linolenic acid (CLA), L-carnitine and acetyl-L-carnitine or others as listed above. CLA is typically provided in amounts of between 5 and 10 g/kg of the diet, or more specifically, about 6-9 or 7-8 g/kg of the diet. L-carnitine is typically supplied at about 100-1000 mg/kg of the diet, or more specifically, about 200-800, 300-700, 400-600, or about 500 mg/kg of the diet. Acetyl-L-carnitine is typically supplied at about 50-150 mg/kg of the diet, or more specifically, about 60-140, 70-130, 80-120, 90-110, or about 100 mg/kg of the diet.

Formulations s may also contain an anti-glycation agent, such as carnosine (beta-alanyl-L-histidine). Carnosine is typically provided in amounts of between about 100-1000 mg/kg of the diet, or more specifically, about 200-800, 300-700, 400-600, or about 500 mg/kg of the diet.

A formulation referred to herein as "Cocktail II" can provide the following in a diet: chromium tripicolinate, 0.5 mg/kg; grape seed extract, 250 mg/kg; zinc monomethionate, 78 mg/kg; CLA (65%), 5000 mg/kg; carnitine, 400 mg/kg; acetyl-carnitine, 100 mg/kg and carnosine, 500 mg/kg. Cocktail II for human consumption can provide the following: chromium picolinate120 µg/day; grape seed extract, 150 mg/day; zinc sulfate 15 mg/day; CLA (65%), 2000 mg/day; carnitine, 2500 mg/day; acetyl-carnitine, 500 mg/day; and carnosine, 500 mg/day.

When administered to animals, a cocktail of this type was shown to markedly decrease body weight and body fat, to levels even greater than CR, to decrease lipid peroxidation in muscle tissue, and to retard a significant percentage of age-related changes in gene expression, as described in detail in the examples.

Another type of formulation may contain functional ingredients to reduce or prevent chronic inflammation. This type of formulation can contain at least one source of omega-3 fatty acids and/or curcumunoids. The source of omega-3 fatty acids can be fish oil. The source can be a combination of purified omega-3 fatty acids, such as, but not limited to eicosapentaenoic and docosahexaenoic acids (EPA and DHA). The curcuminoids may include a purified curcumunoid or may contain a combination of more than one curcumunoid. Curcumunoids include, but are not limited to curcumin (1,7-*bis*-(4-hydroxy-3-methoxyphenyl)-hepta-1,6-diene-3,5-dione; 1-(4-hydroxyphenyl)-7-(4-hydroxy-3-methoxyphenyl)-hepta-1,6-diene-3,5-dione; 1,7-*bis*-(4-hydroxyphenyl)-hepta-1,6-diene-3,5-dione), demethoxycurcumin and bisdemethoxycurcumin.

Fish oil can be provided within the following ranges (g/kg of the diet): 10-50, 15-40, 20-30, orabout 26 g/kg of the diet. Curcumunoids are provided within the following ranges (mg/kg of the diet): 100-1,000, 200-900, 300-750, 400-600, orabout 500 mg/kg of the diet. A formulation referred to herein as "Cocktail III" can provide the following in a diet: fish oil, 26.5 g/kg; and cucurmin extract, 500 mg/kg of the diet.

Other combinations may also be formulated. For example, an antioxidant-rich formulation may be combined with a mixed function formulation, as would be exemplified by a combination of Cocktail I with Cocktail II. Alternatively, an antioxidant-rich formulation may be combined with an anti-inflammatory formulation, as would be exemplified by a combination of Cocktail I with Cocktail III, or a combination of all three Cocktails. Another alternative may comprise an antioxidant-rich formulation combined with a mixed function formulation and an anti-inflammatory formulation, as would be exemplified by a combination of Cocktail I with Cocktail II and Cocktail III.

The composition can be a dietary supplement, such as a gravy, drinking water, beverage, yogurt, powder, granule, paste, suspension, chew, morsel, treat, snack, pellet, pill, capsule, tablet, or any other delivery form. The dietary formulations can be incorporated into human and pet food compositions. These will advantageously include foods intended to supply necessary dietary requirements, as well as treats (*e.g.,* biscuits) or other dietary supplements. Optionally, the food compositions can be a dry composition (for example, kibble), semi-moist composition, wet composition, or any mixture thereof. The dietary supplement can comprise a high concentration of ingredients that improve longevity, such that the supplement can be administered to the animal in small amounts, or in the alternative, can be diluted before administration to an animal. The dietary supplement may require admixing with water prior to administration to the animal.

The compositions may be refrigerated or frozen. The ingredients that improve longevity may be pre-blended with the other components of the composition to provide the beneficial amounts needed, may be coated onto a pet food composition, or may be added to the composition prior to offering it to the animal, for example, using a sprinkled powder or a mix.

The dietary formulations and compositions can optionally comprise supplementary substances such as minerals, vitamins, salts, condiments, colorants, and preservatives. Non-limiting examples of supplementary minerals include calcium, phosphorous, potassium, sodium, iron, chloride, boron, copper, zinc, manganese, iodine, selenium and the like. Non-limiting examples of supplementary vitamins include vitamin A, various B vitamins, vitamin C, vitamin D, vitamin E, and vitamin K. Additional dietary supplements may also be included, e.g., niacin, pantothenic acid, inulin, folic acid, biotin, amino acids, and the like.

Pet food or pet treat compositions can comprise, on a dry matter basis, from about 15% to about 50% crude protein, by weight of the composition. The crude protein material may comprise vegetable proteins such as soybean, cottonseed, and peanut, or animal proteins such as casein, albumin and meat protein. Non-limiting examples of meat protein useful herein include pork, lamb, equine, poultry, fish, and mixtures thereof.

The dietary formulations and compositions may further comprise, on a dry matter basis, from about 5% to about 40% fat, by weight of the composition. The compositions may further comprise a source of carbohydrate. The compositions may comprise, on a dry matter basis, from about 15% to about 60% carbohydrate, by weight of the composition. Non-limiting examples of such carbohydrates include grains or cereals such as rice, corn, sorghum, alfalfa, barley, soybeans, canola, oats, wheat, and mixtures thereof. The compositions may also optionally comprise other materials such as dried whey and other dairy by-products.

The dietary formulations and compositions may also comprise at least one fiber source. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide additional to the short chain oligofructose, mannanoligofructose, soy fiber, arabinogalactan, galactooligosaccharide, arabinoxylan, or mixtures thereof. Alternatively, the fiber source can be a fermentable fiber. Fermentable fiber has previously been described to provide a benefit to the immune system of a companion animal. Fermentable fiber or other compositions known to those of skill in the art which provide a prebiotic composition to enhance the growth of probiotic microorganisms within the intestine may also be incorporated into the composition to aid in the enhancement of the benefit to the immune system of an animal. Additionally, probiotic microorganisms, such as *Lactobacillus* or *Bifidobacterium* species, for example, may be added to the composition.

The dietary formulation or composition can be a complete and nutritionally balanced pet food. In this context, the pet food may be a wet food, a dry food, or a food of intermediate moisture content, as would be recognized by those skilled in the art of pet food formulation and manufacturing. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15%, and therefore is presented, for example, as small biscuit-like kibbles. The compositions, dietary formulations, and dietary supplements may be specially formulated for adult animals, or for older or young animals, for example, a "puppy chow," "kitten chow," "adult" or "senior" formulation. In general, specialized formulations will comprise energy and nutritional requirements appropriate for animals at different stages of development or age.

Certain aspects can be preferably used in combination with a complete and balanced food (for example, as described in National Research Council, 1985, Nutritional Requirements for Dogs, National Academy Press, Washington D.C., or Association of American Feed Control Officials, Official Publication 1996). That is, dietary formulations or compositions comprising at least three ingredients that improve longevity by mimicking at least one longevity-promoting effect of caloric restriction can be used with a high-quality commercial food. As used herein, "high-quality commercial food" refers to a diet manufactured to produce the digestibility of the key nutrients of 80% or more, as set forth in, for example, the recommendations of the National Research Council above for dogs, or in the guidelines set forth by the Association of American Feed Control Officials. Similar high nutrient standards would be used for other animals.

The skilled artisan will understand how to determine the appropriate amount of longevity-enhancing ingredients to be added to a given dietary formulation or composition. Such factors that may be taken into account include the type of composition (*e.g.,* pet food composition versus dietary supplement), the average consumption of specific types of compositions by different animals, and the manufacturing conditions under which the composition is prepared. Preferably, the concentrations of a given longevity-enhancing ingredient to be added to the composition are calculated on the basis of the energy and nutrient requirements of the animal. The longevity-enhancing ingredients can be added at any time during the manufacture and/or processing of the composition. This includes, without limitation, incorporation within the formulation of the pet food composition or dietary supplement, or as a coating applied to the pet food composition or dietary supplement.

The compositions can be made according to any method suitable in the art such as, for example, that described in Waltham Book of Dog and Cat Nutrition, Ed. ATB Edney, Chapter by A. Rainbird, entitled "A Balanced Diet" in pages 57 to 74, Pergamon Press Oxford.

Disclosed are methods for increasing longevity in an animal, including humans, comprising administering to the animal a dietary formulation or composition comprising at least three ingredients that enhance longevity, each ingredient being from a different one of five categories of ingredients that improve longevity by mimicking at least one longevity-promoting effect of caloric restriction, wherein the categories are antioxidants, anti-glycation agents, reducers of body weight or body fat, promoters of high insulin sensitivity or low blood insulin or blood glucose, and anti-inflammatory agents, in an amount effective to enhance longevity in the animal. The composition can be a pet food composition or a dietary supplement, as exemplified herein. Animals may include any domesticated or companion animals as described above. The animal can be a companion animal such as a dog or cat. The composition can be a food or dietary supplement formulated for human consumption, and is administered to, or consumed by, a human for the purpose of enhancing longevity. The formulation is administered on a regular basis, which can be at least once daily. The formulation can be administered as part of a daily dietary regimen for at least about one week, or at least about one month, or at least about three months or longer, up to the duration of the animal's life.

The compositions can be administered to the subject by any of a variety of alternative routes of administration. Such routes include, without limitation, oral, intranasal, intravenous, intramuscular, intragastric, transpyloric, subcutaneous, rectal, and the like. Preferably, the dietary formulations or compositions are administered orally. As used herein, the term "oral administration" or "orally administering" means that the subject ingests, or a human is directed to feed, or does feed, an animal one or more of the inventive compositions described herein.

Wherein the human is directed to feed the composition to an animal, such direction may be that which instructs and/or informs the human that use of the composition may and/or will provide the referenced benefit, for example, the enhancement of cognitive function in the animal. Such direction may be oral direction (*e.g*., through oral instruction from, for example, a physician, veterinarian, or other health professional, or radio or television media (*i.e.,* advertisement), or written direction (*e.g*., through written direction from, for example, a physician, veterinarian, or other health professional (*e.g.,* prescriptions), sales professional or organization (*e.g.,* through marketing brochures, pamphlets, or other instructive paraphernalia), written media (*e.g.,* internet, electronic mail, or other computer-related media), and/or packaging associated with the composition (*e.g.,* a label present on a container holding the composition).

Administration can be on an as-needed or as-desired basis, for example, once-monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the animal or otherwise contacted with or admixed with daily feed or food. When utilized as a daily feed or food, administration will be well known to those of ordinary skill.

Administration can also be carried out as part of a diet regimen in the animal. For example, a diet regimen may comprise causing the regular ingestion by the animal of a composition comprising at least three ingredients that improve longevity, in an amount effective to increase longevity in the animal. Regular ingestion can be once a day, or two, three, four, or more times per day, on a daily basis. The goal of regular ingestion is to provide the animal with the preferred daily dose of the ingredients that improve longevity, as exemplified herein.

The daily dose of compositions can be measured in terms of grams of antioxidants, anti-glycation agents, reducers or body weight or body fat, promoters of high insulin sensitivity or low blood insulin or low blood glucose, or anti-inflammatory agents per kg of body weight (BW) of the animal, as exemplified herein.

According to the methods disclosed herein, administration of the compositions , including administration as part of a diet regimen, can span a period of time ranging from gestation through the adult life of the animal.

The following examples are provided to describe the invention in greater detail. They are intended to illustrate, not to limit, the invention.

### Example 1

The feeding protocol was eleven months in duration. Fifteen month-old mice [C57B1/6] were fed 24 g/wk[AIN-93M - American Institute of Nutrition (AIN) purifed diet formula for maintenance of mature rodents] (except for the calorie-restricted group as specified below, which were fed 18 g/wk for eleven months. Treatments consisted of supplementation to the basic feeding protocol with one or more of the following three cocktails:

### Cocktail I:

| **Compound** | **Dose (mg/kg diet)** |
|---|---|
| d-alpha tocopherol | 500 |
| Natural mixed carotenoids | 50 |
| Selenomethionine (39% selenium) | 0.2 selenium |
| Ascorbic acid (vitamin C) | 450 |
| Lycopene | 50 |

### Cocktail II:

| **Compound** | **Dose (mg/kg diet unless otherwise stated)** |
|---|---|
| Chromium tripicolinate | 0.5 |
| Grape seed extract | 250 |
| Zinc monomethionate | 15 mg/kg elemental zinc (78 mg/kg Zn methionine) |
| CLA (65%) | 7.7 g/kg |
| L-carnitine | 490 |
| Acetyl-L-carnitine | 103 |
| Carnosine | 500 |

### Cocktail III:

| **Compound** | **Dose (mglkg diet unless otherwise stated)** |
|---|---|
| Fish oil | 26.5 g/kg |
| Cucurmin extract | 500 |

The protocol design was as follows:

| **Group** | **Nickname** | **Size (n)** | **Treatment** |
|---|---|---|---|
| 1 | 201 LA (Diet A) | 15 | Cocktail I |
| 2 | 201 LB (Diet B) | 15 | Cocktails I and II |
| 3 | 201 LC (Diet C) | 15 | Cocktails I and III |
| 4 | 201 LD (Diet D) | 15 | Cocktails I, II and III |
| 5 | 201 LE (Diet E) | 15 | No cocktail (negative control) |
| 6 | 201 LF (Diet F) | 15 | Calorie restriction (CR) (positive control) |

At the completion of the eleven-month feeding protocol, all animals that survived were sacrificed. Assessments were made of phenotypic features, biochemical parameters and gene expression profiles from muscle, adipose tissue, and lymphocyte, as described in the examples to follow. Muscle was selected as a sample source because it is a post-mitotic tissue in which cells will not renew. As such this tissue should reflect aging-related damage and associated changes in gene expression. Lymphocytes were selected as an alternative sample source due to the accessibility of this tissue without the use of invasive procedures such as biopsy. Adipose tissue was examined because of the pronounced effect of certain of the treatments, namely diets containing Cocktail II, on fat pad content of the mice.

### Example 2

Body weights of animals were measured weekly during the eleven month protocol. Results are shown in Figure 1. As can be seen, the highest overall body weights were maintained by the control group (Diet E), with similar body weight maintenance by Diet A (Cocktail I) and Diet C (Cocktail I and III). A pronounced initial drop in body weight was seen in Diet F animals (CR); however, by the end of the protocol, similarly reduced weights were seen in animals fed Diets B (Cocktail I and II), D (Cocktail I, II and III) and F (CR).

Figure 2 shows changes in body weight, stripped carcass weight and fat pad weight of the animals over eleven months of the feeding protocol. The largest changes were observed in animals fed Diets B (Cocktail I and II), D (Cocktail I, II and III) and F (CR). Most of those observed changes were due to decreases in fat pad weight (Figure 2, bottom panel).

The survival rates of the animals on the protocol are shown in Table 2-1 below.

**Table 2-1**

| | Diet A (Cocktail I) | Diet B (Cocktail I and II) | Diet C (Cocktail I and III) | Diet D (Cocktail I, II and III) | Diet F (CR) | Diet E (no Treatment) |
|---|---|---|---|---|---|---|
| # mice at | 15 | 15 | 15 | 15 | 15 | 15 |
| beginning | | | | | | |
| # mice at 11 months | 10 | 11 | 9 | 9 | 12 | 7 |
| Survival rate | 66.7% | 73.3% | 60.0% | 60.0% | 80.0% | 46.7% |

**Summary:** In this feeding protocol, nutrient blends containing cocktail II resulted in significantly lower body weight and body fat compared with control mice and all other treatments, including CR. Lean body mass was similar to that of CR treated mice.

CR resulted in the highest survival rate (80%), followed by Diet B (cocktails I+II, 73%). Control mice had the lowest survival rate (46%). Due to small sample size, it was not determined whether CR or cocktails I+II had statistically significant impact on longevity.

### Example 3

Because lipid peroxidation is an indicator of oxidative stress in cells and tissues, the effects of CR and the various diets on lipid peroxidation in muscle were assessed. Levels of fatty acid peroxidation byproducts malondialdehyde (MDA) and 4-hydoxyalkenals (4-HDA) were determined in the muscle from mice that consumed cocktail Diets A-D, as well as in young (5 months old) and old mice (26 months old) fed the AIN-93M control diet and mice on the CR diet (Diet F). As shown in Figure 3, Mice fed cocktail Diet C (Cocktail I + III) were found to exhibit high levels of lipid peroxidation. The levels of lipid peroxidation in these mice closely approximated the levels observed in old mice fed the AIN-93M control diet. In contrast, animals fed Diets A (Cocktail I), B (Cocktail I + II, p<0.05), and D (Cocktail I + II + III, p<0.05) demonstrated lower levels of lipid peroxidation relative to the old mice. Indeed, the lipid peroxidation levels in mice consuming Diets A, B, and D most closely approximated the levels of peroxidation observed in young mice. Of note, mice fed Diets A, B, and D were found to exhibit lower levels of lipid peroxidation than mice fed the CR Diet, and Diets B (P<0.05) and D (p<0.05) produced lower levels of lipid peroxidation than the levels observed in young mice. Diets A, B (p<0.05), and D (p<0.05) produced lower levels of lipid peroxidation relative to the CR mice, and Diets B (p<0.05) and D (p<0.05) produced lower levels of lipid peroxidation relative to the young mice.

### Example 4

Microarray analyses were carried out to determine genes that were significantly affected by aging in muscle, and to determine the effects of caloric restriction and the various nutrient blends on the expression of such genes. Affymetrix GeneChip® Mouse Expression Set 430A (Affymetrix, Inc., Santa Clara, CA), containing sequence clusters created from the UniGene database (Build 107, June 2002, National Center for Biotechnology Information) were analyzed using Affymetrix GeneChip® Operating Software. The data were normalized, and background was subtracted from the analyses.

Genes subject to the microarray data analysis were selected according to the following criteria: 1) genes that were not detected in young mice (5 months old) were removed; 2) significant differences in signal intensity in young versus old mice, as determined by Student's t test (p value of < 0.05 or < 0.01 (two tailed distribution); and 3) fold changes in signal intensity: ≥ 1.2 and ≤ -1.2 in intensity (corresponding to 20% up- or down-regulation in aged relative to young mice).

The effects of the various diet regimens were then assessed for the selected genes. Mice were fed each of the Diets A-F as described in Example 1. Whether a given diet produced a preventive effect on aging was evaluated in terms of signal intensity on the microarray. The following formula was used to determine the preventive effect of each diet: {100-[(young-treatment) X 100/(young-old)]}.

According to this formula, for a given gene, if the effects observed for a given diet regimen equaled the effects observed in young mice, then the dietary formulation prevented age-induced change in that gene by 100%. If the effects observed for a given diet regimen were higher than the effects observed in young mice, then the dietary formulation prevented more than 100% of the age-associated change in expression of the gene. If the effects observed for a given diet regimen were found to be lower than the effects observed in young mice, but higher than the effects observed in the old mice, then the dietary formulation partially prevented age-induced changes in the expression of the gene. If the effects observed for a given diet regimen were found to be lower than the effects observed in old mice, then the diet regimen was deemed to accelerate age-induced changes in gene expression.

The average change in signal intensity for all genes selected from mouse muscle tissue was calculated for mice fed each experimental diet relative to old mice, and the results are presented in Figure 4. All cocktail diets partially prevented age-related changes in muscle tissue gene expression relative to old mice. Although Diets B (Cocktail I + II) and C (Cocktail I + III) produced an average of slightly less than 30% prevention, Diets A (Cocktail I) and D (Cocktail I + II + III) produced an average of slightly higher than 30% prevention. Animals fed Diet F (CR dietary regimen) were observed to produce a higher than 40% prevention of age-induced changes in gene expression.

The number of muscle tissue genes in which the experimental diets were found to exert a statistically significant effect (p<0.01) are listed below in Table 4-1.

**Table 4-1.**

| **Gene Type** | **Number** |
|---|---|
| Apoptosis Regulatory Proteins | 4 |
| Cell Adhesion Proteins | 12 |
| Cell Cycle/Cell Growth Regulatory Proteins | 23 |
| Chromosome Organization Proteins | 4 |
| Development/Cell Differentiation Proteins | 13 |
| DNA Methylation Proteins | 3 |
| DNA Repair/ DNA Replication Proteins | 7 |
| Energy Metabolism Proteins | 22 |
| Hormone Metabolism Proteins | 5 |
| Inflammatory Response Proteins | 20 |
| General Metabolism Proteins | 10 |
| Neuronal Factors | 3 |
| Protein Phosphorylation/Protein Modification Proteins | 15 |
| Protein Synthesis Proteins | 16 |
| Protein Transport Proteins | 16 |
| RNA Metabolism Proteins | 7 |
| Signal Transduction Proteins | 22 |
| Stress Response Proteins | 30 |
| Structural Proteins | 24 |
| Transcription Factors | 34 |
| Transport Proteins | 16 |
| Other Functions | 17 |
| Unknown Functions | 108 |
| General Effects of Diets (Total number of genes) | 431 |

Changes in the body that lead to aging and aging-related diseases include increased stress-induced apoptosis, increased inflammation, increased oxidative stress, compromised insulin-IGF-1 pathway, and compromised insulin sensitivity. Accordingly, caloric restriction and the various experimental diets described herein were evaluated for their respective effects on specific genes related to these changes.

Figure 5 shows the preventive effects of CR and the dietary cocktails on aging-induced apoptosis gene changes in muscle from mice. All cocktail diets demonstrated a measurable effect on apoptosis-related genes in the muscle tissue relative to old mice.

The effects of CR and the dietary cocktails were also evaluated for specific apoptosis-related genes. As shown in Table 4-2 below, CR and the dietary cocktails exerted preventive effects on aging-induced increase in apoptosis-related genes. Similarly, as shown in Table 4-3, CR and the dietary cocktails exerted preventive effects on aging-induced decrease in apoptosis-related genes.

**Table 4-2. Preventive effects (%) on aging-induced increase in apoptosis-related genes.**

| | **Diet A (I)** | **Diet B (I + II)** | **Diet C (I+III)** | **Diet D (I+II+III)** | **Diet F (CR)** |
|---|---|---|---|---|---|
| **Cyclin L2** (tumor cell growth inhibition, apoptosis promotion) | 86 | 51 | 48 | 78 | 82 |
| **Delta Sleep Inducing Peptide Immunoreactor** (Dsip 1) | 47 | -29 | 55 | -77 | 86 |
| **Mitogen Activated Protein Kinase Kinase 7** (Map2k7) | 47 | 94 | 70 | 51 | 36 |
| **Bcl-associated death promoter** (Bad) | -23 | 1 | 21 | 87 | 68 |
| **Pleimorphic adenoma gene-like** 1 (Plag1 1) | -6 | 89 | 3 | 115 | 83 |

**Table 4-3. Preventive effects (%) on aging-induced decrease in apoptosis-related genes.**

| | **Diet A (I)** | **Diet B (I + II)** | **Diet C (I+III)** | **Diet D (I+II+III)** | **Diet F (CR)** |
|---|---|---|---|---|---|
| **Clusterin** (sCLU: cytoprotective, nCLU-proapoptosis) | 23 | 23 | -17 | 4 | 55 |
| **B-amyloid binding protein Precursor** | 36 | 29 | 55 | 38 | 25 |

Next, the preventive effects of CR and the dietary cocktails on aging-related stress response gene changes were evaluated in muscle from mice, the results of which are shown in Figure 6. The aging-increased stress response in muscle tissue includes increased expression of inducible heat shock proteins, increased expression of DNA-damage inducible genes, and increased expression of oxidative stress-inducible genes. All cocktail diets demonstrated a measurable effect on aging-related stress response genes in the muscle tissue relative to old mice (Figure 6).

The effects of CR and the dietary cocktails were also evaluated for specific stress response genes in muscle. Table 4-4 shows the preventive effects of CR and the dietary cocktails on the aging-induced increase in heat shock proteins. Table 4-5 shows the preventive effects of CR and the dietary cocktails on the aging-induced increase in DNA damage-inducible genes. Table 4-6 shows the preventive effects of CR and the dietary cocktails on the aging-induced increase in oxidative stress-inducible genes. Table 4-7 shows the preventive effects of CR and the dietary cocktails on the aging-induced increase in stress-related genes generally.

**Table 4-4. Preventive effects (%) of CR and cocktail diets on aging-induced increase in HSP.**

| | **Diet A (I)** | **Diet B (I+II)** | **Diet C (I+III)** | **Diet D (I+II+III)** | **Diet F (CR)** |
|---|---|---|---|---|---|
| **Heat Shock Protein 4** (HSP70) | 24 | 36 | 57 | 31 | 45 |
| **Heat Shock Protein 1, beta** (Hsp84 or Hsp84-1, or Hsp90) | 37 | 54 | 31 | 14 | 41 |
| **Heat Shock Protein 2** (Heat shock 27 kDa protein) | 59 | 34 | 40 | 31 | 56 |

**Table 4-5. Preventive effects (%) of CR and cocktail diets on aging-induced increase in DNA damage-inducible genes.**

| | **Diet A (I)** | **Diet B (I + II)** | **Diet C (I+III)** | **Diet D (I+II+III)** | **Diet F (CR)** |
|---|---|---|---|---|---|
| **PRP19/PSO4 homolog** (DSB DNA repair) | 38 | 75 | 45 | 69 | 8 |
| **Damage specific DNA binding proteins 1** (Ddbp1) | 15 | 8 | -6 | -16 | 42 |
| **Damage specific DNA binding protein 2** (Ddbp2) (global genomic repair/damage recognition/mismatch repair/tumor suppressor | 76 | 54 | 12 | 96 | 46 |
| **Nuclear Factor I/C** (DNA replication) | 149 | 69 | -146 | 5 | 96 |

**Table 4-6. Preventive effects (%) of CR and cocktail diets on aging-induced increase in oxidative stress-inducible genes.**

| | **Diet A (I)** | **Diet B (I + II)** | **Diet C (I+III)** | **Diet D (I+II+III)** | **Diet F (CR)** |
|---|---|---|---|---|---|
| **Glutatione Peroxidase 4** (PHGPx) | 34 | 36 | 25 | 61 | 53 |
| **Peroxiredoxin** 1 (Thioredoxin peroxidase 2) | 59 | 44 | 30 | 21 | 52 |
| **Thioredoxin Interacting Protein** | 95 | 82 | 82 | 58 | 123 |
| **Glutathione Reductase 1** (Gsr) | 47 | 25 | 46 | 33 | 54 |
| **Xanthine Dehydrogenase** | 61 | 41 | 75 | 79 | 72 |
| **Mitogen Activated Protein Kinase Kinase** (Map2k7) | 47 | 94 | 70 | 51 | 36 |

**Table 4-7. Preventive effects (%) of CR and cocktail diets on aging-induced increase in stress-related genes.**

| | **Diet A (I)** | **Diet B (I + II)** | **Diet C (I+III)** | **Diet D (I+II+III)** | **Diet F (CR)** |
|---|---|---|---|---|---|
| **Cold Inducible RNA Binding Protein** (cold-induced suppression of cell proliferation) | 50 | 77 | 13 | 60 | 99 |
| **Peroxisomal Biogenesis Factor 11b** (peroxisome organization and biogenesis) | 66 | 64 | 61 | 38 | 92 |
| **Small Glutamine-Rich Tetratricopeptide Repeat (TPR)-containing, alpha** (cochaperone that binds HSC70 and HSP70 and regulates ATPase activity) | 78 | 33 | -50 | -25 | 99 |

Next, the preventive effects of CR and the dietary cocktails on aging-related inflammatory response gene changes were evaluated in muscle from mice, the results of which are shown in Figure 7. All cocktail diets demonstrated a measurable effect on aging-related stress response genes in the muscle tissue relative to old mice.

The effects of CR and the dietary cocktails were also evaluated for specific inflammatory response genes in muscle. As shown in Table 4-8 below, CR and the dietary cocktails exerted preventive effects on aging-induced increase in inflammation/immune-related genes. Similarly, as shown in Table 4-9, CR and the dietary cocktails exerted preventive effects on aging-induced decrease in inflammation/immune-related genes.

**Table 4-8. Preventive effects (%) of CR and cocktail diets on aging-induced increase in inflammation/immune-related genes.**

| | **Diet A (I)** | **Diet B (I + II)** | **Diet C (I+III)** | **Diet D (I+II+III)** | **Diet F(CR)** |
|---|---|---|---|---|---|
| **Ubiquitin Thiolesterase Protein** (OTUB1) | 89 | 11 | 3 | -49 | 80 |
| **Core Promoter Element Binding Protein** | 46 | 14 | 50 | 24 | 79 |
| **CD59a Antigen** (potent inhibitor of the complement membrane attack complex action) | 49 | 78 | 92 | 110 | 54 |

**Table 4-9. Preventive effects (%) of CR and cocktail diets on aging-induced decrease in inflammation/immune-related genes.**

| | **Diet A (I)** | **Diet B (I + II)** | **Diet C (I+III)** | **Diet D (I+II+III)** | **Diet F (CR)** |
|---|---|---|---|---|---|
| **Interferon Consensus Sequence Binding Protein 1** (Icsbp1) | 24 | 23 | 4 | 12 | 22 |
| **Interleukin 16** | 30 | 26 | -8 | -2 | -15 |
| **CD790B Antigen** | 43 | 66 | -14 | 31 | -20 |
| **Small Inducible Cytokine B13 Precursor** (Cxcl13) | 116 | 116 | -12 | 120 | 98 |
| **CD79A Antigen** (CD79a) | 39 | 70 | -7 | 39 | -7 |
| **Fc Receptor, IgE, low affinity II, alpha polypeptide** | -6 | 14 | -8 | 2 | -20 |
| **Complement Receptor 2** | 41 | 38 | -48 | -10 | -10 |
| **Uteroglobin-Related Protein 2 Precursor** (secretoglobin family 3A, member 1, anti-inflammatory protein) | 5 | 25 | 31 | 46 | 36 |
| **Polymeric Immunoglobulin Receptor** | -46 | 7 | -38 | -10 | 32 |

The effects overall effects of age, CR, and the various dietary formulation described herein on the expression of insulin receptor substrate 1 (IRS-1) were also evaluated. IRS-1 signal intensities were determined in the microarray for mouse muscle tissue in mice fed each of the cocktail diets and in mice fed a caloric restriction dietary regimen, and were compared to IRS-1 signal intensities in muscle tissue from control young and old mice (Figure 8). Mice fed cocktail Diets A (I), C (I + III), and D (I + II + III) showed the lowest signal intensities for IRS-1, which were only slightly above the signal intensities for IRS-1 observed in control old mice. Mice fed cocktail Diet B (I + II) showed the highest signal intensity among the cocktail diets, which was only slightly below the signal intensity observed in young controls. Diet F (CR) mice demonstrated the highest overall signal intensity, which was determined to be higher than the signal intensity observed in the young control mice.

The preventive effects of CR and the dietary cocktails on aging-induced reduction of muscle IRS-1 expression were evaluated in muscle from mice, as shown in Figure 9. Consistent with the results observed for the IRS-1 signal intensity (Figure 8), mice fed cocktail Diets A (I), C (I + III), and D (I + II + III) showed the lowest preventive effects against aging-induced reduction in IRS-1 expression (Figure 9). Mice fed cocktail B (I + II) demonstrated the strongest preventive effects against the reduction in IRS-1 expression among the cocktail diets tested. Mice fed the CR dietary regimen demonstrated a significantly higher preventive effect relative to the cocktail-fed mice, which in fact was a higher than the effect observed in young controls.

***Summary:*** Caloric restriction exerted higher than 40% prevention of age-induced changes in gene expression and partially retarded some of the aging-induced changes in many pathways that are involved in the aging process and ageing-related diseases, for instance, apoptosis genes, stress-related genes, DNA repair, and inflammation-related genes expression, and completely prevented the aging-induced decrease in expression of insulin signaling-related gene in mouse muscle tissue. All cocktail diets also partially prevented age-related changes in muscle tissue gene expression relative to old mice. Diets B (Cocktail I + II) and C (Cocktail I + III) produced an average of slightly less than 30% prevention, Diets A (Cocktail I) and D (Cocktail I + II + III) produced an average of slightly higher than 30% prevention. In addition, the nutrient blends described herein partially reversed some of the aging-induced changes in many pathways that are involved in the aging process and ageing-related diseases, for instance, apoptosis genes, stress-related genes, DNA repair, and inflammation-related genes expression. Cocktail I alone demonstrated some preventive effect on the aging-induced decrease of IRS-1 expression. Cocktails I + II demonstrated higher preventive effects on the aging-induced decrease in IRS-1 expression than cocktail I alone.

### Example 5

Microarray analyses were carried out to determine genes that were significantly affected by aging in lymphocytes, and to determine the effects of caloric restriction and the various nutrient blends on the expression of such genes. Affymetrix GeneChip® Mouse Expression Set 430A (Affymetrix Inc., Santa Clara, CA), containing sequence clusters created from the UniGene database (Build 107, June 2002 (National Center for Biotechnology Information) were analyzed using Affymetrix GeneChip^{g} Operating Software, as described in Example 4. Genes subject to the microarray data analysis were selected according to the criteria set forth in Example 4, as was the assessment of the effects of the various diet regimens on the selected genes.

The average change in signal intensity for all genes selected from mouse muscle tissue was calculated for mice fed each experimental diet relative to old mice, and the results are presented in Table 5-1.

**Table 5-1. Prevention (%) of aging-related changes in gene expression in lymphocytes by CR or diet.**

| Function | (# of Genes Affected) | Diet A (I) Old | Diet B (I+II) / Old | Diet C (I+III)/ Old | Diet D (I+II+III)/Old | Diet F CR / Old |
|---|---|---|---|---|---|---|
| Cell cycle/ cell growth | (7) | 43 | 45 | 49 | 94 | 42 |
| Protein biosynthesis | (11) | 26 | 22 | 14 | 24 | 24 |
| Protein transport | (8) | 27 | 28 | 21 | 30 | 35 |
| RNA metabolism | (13) | 43 | 62 | 51 | 55 | 48 |
| Signal transduction | (11) | 33 | 33 | 32 | 22 | 37 |
| Unknown | (37) | 41 | 46 | 39 | 45 | 37 |
| Total | (127) | 37 | 42 | 36 | 43 | 37 |

As can be seen from Table 5-1, all cocktail diets, as well as CR, prevented age-related changes in lymphocyte gene expression relative to old mice. Diets A, (Cocktail I), C (Cocktail I + III) and F (CR) produced an average of slightly less than 40% prevention, Diets B (Cocktail I + II) and D (Cocktail I + II + III) produced an average of slightly higher than 40% prevention.

### Example 6

Microarray analyses were carried out to determine genes that were significantly affected by aging in adipose tissue, and to determine the effects of caloric restriction and the various nutrient blends on the expression of such genes. Affymetrix GeneChip® Mouse Expression Set 430A (Affymetrix Inc., Santa Clara, CA), containing sequence clusters created from the UniGene database (Build 107, June 2002, National Center for Biotechnology Information) were analyzed using Affymetrix GeneChip® Operating Software, as described in Example 4. Genes subject to the microarray data analysis were selected according to the criteria set forth in Example 4, as was the assessment of the effects of the various diet regimens on the selected genes.

Figure 10 shows a summary of age-related changes in adipose tissue gene expression. As can be seen, 643 genes, representing a variety of different known and unknown functions, exhibited altered levels of expression in old mice as compared with young mice (p<0.01).

The influence of CR or dietary regimen on age-related gene expression in adipose tissue is shown in Figure 10 and Table 6-1.

**Table 6-1. Summary of Dietary Influences on Age-Related Changes in Gene Expression in Adipose Tissue (at p<0.01 and p<0.05)**

| Function | Aging | CR 0.01 / 0.05 | DIET A 0.01 / 0.05 | DIET B 0.01 / 0.05 | DIET C 0.01 / 0.05 | DIET D 0.01 / 0.05 |
|---|---|---|---|---|---|---|
| AMINO ACID METABOLISM | 6 | 0 / 1 | 2 / 2 | 2 / 3 | 2 / 3 | 1 / 2 |
| ANGIOGENESIS | 5 | 2 / 3 | 0 / 0 | 0 / 3 | 0 / 1 | 0 / 2 |
| APOPTOSIS | 25 | 6 / 11 | 3 / 10 | 4 / 8 | 4 / 8 | 2 / 8 |
| CELL ADHESION | 17 | 4 / 5 | 1 / 5 | 5 / 8 | 3 / 4 | 2 / 6 |
| CELL GROWTH AND /OR MAINTENANCE | 36 | 10 / 15 | 3 / 11 | 6 / 10 | 5 / 15 | 9 / 13 |
| CELL PROLIFERATION | 27 | 6 / 11 | 3 / 6 | 6 / 13 | 1 / 4 | 3 / 8 |
| ELECTRON TRANSPORT AND ATP SYNTHESIS | 3 | 0 / 0 | 0 / 0 | 0 / 2 | 0 / 0 | 0 / 0 |
| EXTRACELLULAR MATRIX REMODELING | 5 | 0 / 1 | 0 / 0 | 1 / 1 | 0 / 0 | 0 / 1 |
| IMMUNE RESPONSE AND INFLAMMATION | 32 | 11 / 15 | 3 / 7 | 6 / 16 | 3 / 6 | 5 / 9 |
| METABOLISM, CARBOHYDRATE METABOLISM, | 10 | 2 / 3 | 1 / 2 | 2 / 3 | 0 / 1 | 3 / 4 |
| FATTY ACID | 3 | 0/2 | 0 / 1 | 2 / 2 | 0 / 1 | 2 / 2 |
| METABOLISM, LIPID | 17 | 1 / 6 | 2 / 3 | 5 / 7 | 0 / 3 | 5 / 8 |
| NUCLEIC ACID METABOLISM | 16 | 6 / 8 | 3 / 6 | 1 / 7 | 3 / 6 | 1 / 1 |
| PROTEIN DEGRADATION | 7 | 2 / 4 | 5 / 7 | 0 / 1 | 3 / 5 | 0 / 3 |
| PROTEIN METABOLISM | 28 | 9 / 19 | 4 / 9 | 6 / 10 | 6 / 9 | 6 / 11 |
| PROTEIN MODIFICATION | 11 | 3 / 4 | 5 / 8 | 2 / 5 | 4 / 6 | 2 / 2 |
| PROTEIN SYNTHESIS | 6 | 0 / 2 | 2 / 3 | 1 / 4 | 1 / 1 | 0 / 1 |
| RESPONSE TO EXTERNAL STIMULUS | 5 | 0 / 0 | 1 / 1 | 1 / 1 | 1 / 2 | 1 / 2 |
| RESPONSE TO STRESS | 20 | 6 / 9 | 2 / 6 | 3/8 | 1 / 4 | 4 / 6 |
| SIGNAL TRANSDUCTION | 55 | 15 / 24 | 11 / 18 | 11 / 20 | 8 / 18 | 10 / 14 |
| TRANSCRIPTION | 62 | 12 / 30 | 6 / 8 | 9 / 19 | 6 / 10 | 7 / 17 |
| TRANSPORT | 46 | 17 / 24 | 13 / 18 | 10 / 18 | 8 / 18 | 9 / 16 |
| UNKNOWN | 118 | 25 / 51 | 16 / 36 | 20 / 46 | 18 / 39 | 22 / 43 |

As can be seen from Figure 11, CR and each of Diets A-D (and a combination) prevented certain percentages of the observed age-related changes in gene expression. The largest influence was observed with CR; however, significant influences were also observed with each of the Diets tested. Table 6-1 provides a breakdown of the influenced gene by function.

Figures 12-16 show different analysis of the data. In these analyses, the influence of CR or each of the four Diets on age-related changes in expression of particular genes was plotted. Only genes having an age-related change in expression and a CR or diet-related change in expression are shown. The X axis of each plot represents the fold increase or decrease in expression of a gene in old versus young mice. The Y axis of each plot represents the fold increase or decrease in gene expression of that gene as a result of the treatment (CR or one of Diets A-D). Thus, for example, if a particular gene exhibits a tenfold increase in expression in old versus young mice, and exhibits a six-fold decrease in expression as a result of the dietary treatment, that treatment is said to prevent or reverse the age-related change in expression of that particular gene. Accordingly, the upper left and lower right quadrants of each plot shown in Figures 12-16 represent genes whose age-related change in expression can be prevented, at least in part, by a dietary intervention. By contrast, the upper right and lower left quadrants of each plot shown in Figures 12-16 represent genes whose age-related change in expression is likely not influence by the dietary intervention.

As can be seen from Figures 12-16, of the number of genes whose expression was affected by aging and by the respective dietary treatments, a vast majority of the age-related changes were prevented or reversed, to a varying extent, by that dietary treatment. These results ranged from 68% (Diet D, Cocktails I + II + III] to 97% (CR).

To summarize the data presented above, it was observed that CR prevented the greatest number of age-associated changes in adipose tissue gene expression. Diets A, B, C and D also opposed the development of many age-associated changes in gene expression. As one example, it is noted that *Pltp* expression was increased by all diets, possibly due to the influence of Cocktail I, which was present in all diets.

The protein CD59a is known to be a regulator of the membrane attack complex (complement cascade). The expression of this gene in old versus young mice, and as influenced by the dietary regimens, was examined. Results are shown in Figure 17. As can be seen from the Figure, expression of this gene increased in aged subjects by 1.6 fold as compared with young subjects. Notably, CR and each of Diets A-D were able to decrease expression of this gene as compared with the "old" control and, in some cases, even below the value observed in the "young" control.

## Claims

1. A dietary formulation comprising five categories of ingredients that improve longevity by mimicking at least one longevity-promoting effect of caloric restriction, wherein the categories are:
(a) antioxidants;
(b) anti-glycation agents;
(c) reducers of body weight or body fat;
(d) promoters of high insulin sensitivity or low blood insulin or blood glucose; and
(e) anti-inflammatory agents.

2. The formulation of claim 1, wherein the antioxidants are water-soluble; optionally wherein the water-soluble antioxidants include one or more of Vitamin C, polyphenols, proanthocyanidins, anthocyanins, bioflavonoids, a source of selenium, alpha-lipoic acid, glutathione, catechin, epicatechin, epigallocatechin, epigallocatechin gallate, epicatechin gallate or cysteine; optionally wherein the source of selenium is at least one of sodium selenite, sodium selenate or L-selenomethionine.

3. The formulation of claim 1, wherein the antioxidants are fat-soluble; optionally wherein the fat-soluble antioxidants include one or more of Vitamin E, gamma tocopherol, alpha-carotene, beta-carotene, lutein, zeaxanthin, retinal, astaxanthin, cryptoxanthin, natural mixed carotenoids, lycopene or resveratrol.

4. The formulation of claim 1, containing fat-soluble and water-soluble antioxidants; optionally wherein the antioxidants include Vitamin E, Vitamin C, natural carotenoids, a source of selenium, and lycopene.

5. The formulation of claim 1, wherein the anti-glycation agents include one or more of carnosine or aminoguanidine.

6. The formulation of claim 1, wherein the reducers of body weight or body fat include one or more of conjugated linoleic acid, L-carnitine, acetyl-L-carnitine, pyruvate, polyunsaturated fatty acids, medium chain fatty acids, medium chain triglycerides, or soy isoflavones and their metabolites.

7. The formulation of claim 1 , wherein the promoters of high insulin sensitivity or low blood insulin or blood glucose include one or more of a source of chromium, cinnamon, cinnamon extract, polyphenols from cinnamon and witch hazel, coffee berry extract, chlorogenic acid, caffeic acid, a source of zinc, or grape seed extract.

8. The formulation of claim 1, wherein the anti-inflammatory agents include one or more of a source of omega-3 fatty acids or a source of curcumin; optionally wherein (a) the source of omega-3 fatty acid is at least one of α-linolenic acid, eicosapentaenoic acid, docosapentaenoic acid, docosahexaenoic acid, flax seed, flax oil, walnuts, canola oil, wheat germ, or fish oil: or (b) the source of curcumin is (1,7-bis-(4-hydroxy-3-methoxyphenyl)-hepta-1,6-diene-3,5-dione; 1-(4-hydroxyphenyl)-7-(4-hydroxy-3-methoxyphenyl)-hepta-1,6-diene-3,5-dione; 1,7-bis-(4-hydroxyphenyl)-hepta-1,6-diene-3,5-dione), demethoxycurcumin, or bisdemethoxycurcumin.

## Patentansprüche

1. Nahrungsrezeptur mit fünf Kategorien von Zutaten, die die Langlebigkeit verbessern, indem mindestens eine Langlebigkeit fördernde Wirkung der Kalorienbeschränkung nachgeahmt wird, wobei die Kategorien die folgenden sind:
(a) Antioxidantien;
(b) Antiglykierungsmittel;
(c) Körpergewicht- oder Körperfettreduzierer;
(d) Promotoren hoher Insulinempfindlichkeit oder eines niedrigen Insulinspiegels im Blut oder niedrigen Blutzuckers; und
(e) entzündungshemmende Mittel.

2. Rezeptur nach Anspruch 1, wobei die Antioxidantien wasserlöslich sind; wahlweise wobei die wasserlöslichen Antioxidantien eines oder mehrere der folgenden aufweisen: Vitamin C, Polyphenole, Proanthocyanidine, Anthocyanine, Bioflavonoide, eine Selenquelle, Alpha-Liponsäure, Glutathion, Catechin, Epicatechin, Epigallocatechin, Epigallocatechingallat, Epicatechingallat oder Cystein; wahlweise wobei die Selenquelle mindestens eines der folgenden ist: Natriumselenit, Natriumselenat oder L-Selenomethionin.

3. Rezeptur nach Anspruch 1, wobei die Antioxidantien fettlöslich sind; wahlweise wobei die fettlöslichen Antioxidantien eines oder mehrere der folgenden aufweisen: Vitamin E, Gamma-Tocopherol, Alpha-Carotin, Beta-Carotin, Lutein, Zeaxanthin, Retinal, Astaxanthin, Cryptoxanthin, natürliche gemischte Carotinoide, Lycopen oder Resveratrol.

4. Rezeptur nach Anspruch 1, die fettlösliche und wasserlösliche Antioxidantien enthält; wahlweise wobei die Antioxidantien Vitamin E, Vitamin C, natürliche Carotinoide, eine Selenquelle und Lycopen aufweisen.

5. Rezeptur nach Anspruch 1, wobei die Antiglykierungsmittel eines oder mehrere von Carnosin oder Aminoguanidin aufweisen.

6. Rezeptur nach Anspruch 1, wobei die Körpergewicht- oder Körperfettreduzierer eines oder mehrere der folgenden aufweisen: konjugierte Linolsäure, L-Carnitin, Acetyl-L-Carnitin, Pyruvat, mehrfach ungesättigte Fettsäuren, mittelkettige Fettsäuren, mittelkettige Triglyceride oder Soja-Isoflavone und deren Metaboliten.

7. Rezeptur nach Anspruch 1, wobei die Promotoren hoher Insulinempfindlichkeit oder eines niedrigen Insulinspiegels im Blut oder niedrigen Blutzuckers eines oder mehrere der folgenden aufweisen: eine Chromquelle, Zimt, Zimtextrakt, Polyphenole aus Zimt und Hamamelis, Kaffeebeerenextrakt, Chlorogensäure, Kaffeesäure, eine Zinkquelle oder Traubensamenextrakt.

8. Rezeptur nach Anspruch 1, wobei die entzündungshemmenden Mittel eine oder mehrere der folgenden Quellen aufweisen: eine Quelle von Omega-3-Fettsäuren oder eine Curcumin-Quelle; wahlweise wobei (a) die Quelle von Omega-3-Fettsäuren mindestens eines der folgenden ist: eine α-Linolensäure, Eicosapentaensäure, Docosapentaensäure, Docosahexaensäure, Leinsamen, Leinöl, Walnüsse, Rapsöl, Weizenkeime oder Fischöl, oder (b) die Curcumin-Quelle folgendes ist: (1,7-bis-(4-Hydroxy-3-Methoxyphenyl)-Hepta-1,6-Dien-9,5-Dion; 1-(4-Hydroxyphenyl)-7-(4-Hydroxy-3-Methoxyphenyl)-Hepta-1,6-Dien-3,5-Dion; 1,7-bis-(4-Hydroxyphenyl)-Hepta-1,6-Dien-3,5-Dion), Demethoxycurcumin oder Bisdemethoxycurcumin.

## Revendications

1. Formulation alimentaire comprenant cinq catégories d'ingrédients qui améliorent la longévité en imitant au moins un effet de favorisation de longévité de la restriction calorique, dans laquelle les catégories sont :
(a) antioxydants ;
(b) agents anti-glycation ;
(c) réducteurs de poids corporel ou de graisse corporelle ;
(d) promoteurs de sensibilité haute à l'insuline ou d'insulinémie ou glycémie basse ; et
(e) agents anti-inflammatoires.

2. Formulation selon la revendication 1, dans laquelle les antioxydants sont solubles dans l'eau ; dans laquelle facultativement les antioxydants solubles dans l'eau comprennent un ou plusieurs éléments parmi vitamine C, polyphénols, proanthocyanidines, anthocyanines, bioflavonoïdes, une source de sélénium, acide alpha-lipoïque, glutathion, catéchine, épicatéchine, épigallocatéchine, gallate d'épigallocatéchine, gallate d'épicatéchine ou cystéine ; dans laquelle facultativement la source de sélénium est au moins un élément parmi sélénite de sodium, sélénate de sodium ou L-sélénométhionine.

3. Formulation selon la revendication 1, dans laquelle les antioxydants sont liposolubles ; dans laquelle facultativement les antioxydants liposolubles comprennent un ou plusieurs éléments parmi vitamine E, gamma tocophérol, alpha-carotène, bêta-carotène, lutéine, zéaxanthine, rétinal, astaxanthine, cryptoxanthine, caroténoïdes mélangés naturellement, lycopène ou resvératrol.

4. Formulation selon la revendication 1, contenant des antioxydants liposolubles et solubles dans l'eau ; dans laquelle facultativement les antioxydants comprennent vitamine E, vitamine C, caroténoïdes naturels, une source de sélénium, et lycopène.

5. Formulation selon la revendication 1, dans laquelle les agents anti-glycation comprennent un ou plusieurs éléments parmi carnosine ou aminoguanidine.

6. Formulation selon la revendication 1, dans laquelle les réducteurs de poids corporel ou de graisse corporelle comprennent un ou plusieurs éléments parmi acide linoléique conjugué, L-carnitinè, acétyl-L-carnitine, pyruvate, acides gras polyinsaturés, acides gras à chaîne moyenne, triglycérides à chaîne moyenne, ou isoflavones de soja et leurs métabolites.

7. Formulation selon la revendication 1, dans laquelle les promoteurs de sensibilité haute à l'insuline ou d'insulinémie ou glycémie basse comprennent un ou plusieurs éléments parmi une source de chrome, cannelle, extrait de cannelle, polyphénols de cannelle et d'hamamélis, extrait de cerises de café, acide chlorogénique, acide caféique, une source de zinc, ou extrait de pépins de raisin.

8. Formulation selon la revendication 1, dans laquelle les agents anti-inflammatoires comprennent un ou plusieurs éléments parmi une source d'acides gras oméga-3 ou une source de curcumine ; dans laquelle facultativement (a) la source d'acides gras oméga-3 est au moins un élément parmi acide α-linolénique, acide eicosapentaénoïque, acide docosapentaénoïque, acide docosahexaénoïque, graine de lin, huile de lin, noix, huile de canola, germe de blé, ou huile de poisson ; ou (b) la source de curcumine est (1,7-bis-(4-hydroxy-3-méthoxyphényl)-hepta-1,6-diène-3,5-dione; 1-(4-hydroxyphényl)-7-(4-hydroxy-3-méthoxyphényl)-hepta-1,6-diène-3,5-dione; 1,7-bis-(4-hydroxyphényl)-hepta-1,6-diène-3,5-dione), déméthoxycurcumine ou bisdéméthoxycurcumine.
